# EUROPEAN PATENT APPLICATION

(11) **EP 3 626 703 A1**
(43) Date of publication of application: **25.03.2020**
(21) Application number: 17910437.7
(22) Date of filing: 16.05.2017
(51) Int. Cl.: C07C 225/22, C07C 311/21, C07D 211/14, C07D 241/04, A61K 31/136, A61K 31/18, A61K 31/496

(54) **NOVEL COMPOUND AND PHARMACEUTICAL COMPOSITION COMPRISING SAME AS ACTIVE INGREDIENT**

(71) Applicant: Industry-Academic Cooperation Foundation, Yonsei University, Seoul 03722 (KR); Ewha University-Industry Collaboration Foundation, Seoul 03760 (KR); Cha University Industry-Academic Cooperation Foundation, Pocheon-si, Gyeonggi-do 11160 (KR)
(72) Inventor: KIM, Eosu, Seoul 03722 (KR); NAMKOONG, Kee, Seoul 03722 (KR); JEONG, Jihyeon, Seoul 03722 (KR); JANG, Sooah, Seoul 03722 (KR); KWON, Young Joo, Seoul 03760 (KR); JUN, Kyu Yeon, Seoul 03760 (KR); JEON, Kyung Hwa, Seoul 03760 (KR); PARK, Minsun, Seoul 06713 (KR); KIM, Hyunjeong, Seoul 08028 (KR); NA, Younghwa, Seodaemun-gu Seoul 03709 (KR)
(74) Representative: Müller, Christian Stefan Gerd
(86) International application number: PCT/KR2017/005061
(87) International publication number: WO 2018/212368

(57) **Abstract**

The present invention relates to a novel compound and a pharmaceutical composition comprising the same as an active ingredient. The novel compound, which is obtained by means of carrying out chemical modification in a mother structure showing excellent AMP-activated protein kinase (AMPK) activation efficacy and in silico binding capacity, enables effective prevention or treatment of degenerative neurological disorders by means of effectively inducing AMP-activated protein kinase enzyme activation.

## Description

### Technical Field

The present invention relates to a novel compound and a pharmaceutical composition comprising the same as an active ingredient.

### Background Art

As population aging is rapidly progressing around the world, the number of patients with degenerative neurological diseases such as Alzheimer's disease, Parkinson's disease, and stroke tends to increase rapidly. In South Korea, the population aged 65 or older accounted for 12.2% of the total population in 2013. Thus, South Korea has already entered an aging country, and is expected to become a super-aging country in 2030. As such, as the country ages, prevalence of patients with degenerative neurological diseases increases sharply. Therefore, rapid population aging is causing serious social and economic burdens beyond health and medical problems.

Alzheimer's disease (AD) is a degenerative disease which has, as clinical features, slowly progressing memory disorder, multiple cognitive decline, and behavioral disorder. The main neuropathological finding in this disease is neuronal toxicity which occurs as insoluble proteinous substances aggregate and are deposited in the hippocampus and cortex; and typical examples thereof are senile plaque composed of beta-amyloid (Aβ) outside neurons, and neurofibrillary tangle consisting of hyperphosphorylated tau protein inside neurons. Among these, the amyloid hypothesis, which has been in the spotlight for the past 20 years as the main etiology of Alzheimer's disease, emphasizes that the starting point for inducing neuronal dysfunction and neuronal death is accumulation of beta-amyloid. Accumulation of beta-amyloid resulting from over-expression of beta-amyloid by amyloid precursor protein (APP), presensilin 1/2 (PS 1/2), or apolipoprotein E (Apo E) due to genetic or environmental factors, or from decreased clearance of beta-amyloid causes neuronal toxicity.

Meanwhile, AMP-activated protein kinase (AMPK) is a pivotal enzyme that regulates cell energy metabolism. When cell energy decreases, AMPK is activated by detection of an AMP/ATP ratio and helps the cell's energy production (catabolism). On the contrary, when cell energy is sufficient, activity of AMPK is inhibited to increase anabolism. In particular, an energy homeostasis maintenance function of the AMP-activated protein kinase has been linked to not only metabolic promotion but also to cellular protective and anti-inflammatory effects. Recently, there have been reports that the AMP-activated protein kinase increases a clearance rate of beta-amyloid by increasing autophagy and is involved in anti-dementia efficacy. Apart from this, it has been found that upon activation of neuronal AMP-activated protein kinase, neurogenesis increases, resulting in cognitive amelioration and neuronal protective effects. In this context, substances that activate the neuronal AMP-activated protein kinase can be said to be novel-concept new drug candidates that can play a role in cognitive improvement and anti-dementia.

Despite the amyloid hypothesis, for Alzheimer's disease, Parkinson's disease, and the like which belong to the degenerative neurological diseases, pathogenesis thereof is not fully elucidated, and thus drugs which target a neurotransmission process mainly for symptomatic alleviation are only being developed. There is no therapeutic drug for complete cure.

### Technical Problem

An object of the present invention is to provide a novel compound and a preparation method thereof.

Another object of the present invention is to provide a pharmaceutical composition for preventing or treating degenerative neurological diseases, comprising the compound as an active ingredient.

However, the technical problems to be solved by the present invention are not limited to the above-mentioned problems, and other problems that are not mentioned will be clearly understood by those skilled in the art from the following description.

### Solution to Problem

The present inventors have prepared novel compounds, in particular, compounds that activate an AMP-activated protein kinase (AMPK) enzyme, and have found that these compounds remarkably increase activity of the AMP-activated protein kinase enzyme, thereby exerting a therapeutic effect on degenerative neurological diseases. Based on this finding, the present inventors have completed the present invention.

In an embodiment of the present invention, there is provided a compound represented by the following Formula 1: 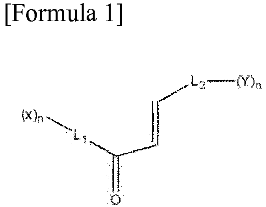 In the Formula 1,
L₁ to L₂ may be each independently selected from the group consisting of C₃ to C₄₀ cycloalkylene, C₆ to C₆₀ arylene, heteroarylene having 5 to 60 nuclear atoms, X and Y may be each independently selected from the group consisting of deuterium, halogen, cyano, nitro, sulfonyl, C₁ to C₁₀ alkylsulfonyl, azide, hydroxy, C₁ to C₄₀ alkyl, C₂ to C₄₀ alkenyl, C₁ to C₄₀ alkoxy, unsubstituted or substituted C₆ to C₆₀ aryloxy, unsubstituted or substituted C₃ to C₄₀ cycloalkyl, unsubstituted or substituted heterocycloalkyl having 3 to 20 nuclear atoms, unsubstituted or substituted C₆ to C₆₀ aryl, unsubstituted or substituted heteroaryl having 5 to 60 nuclear atoms, and -NR'R", R' and R" may be each independently selected from the group consisting of hydrogen, C₁ to C₁₀ alkyl, C₆ to C₆₀ aryl, C₃ to C₄₀ cycloalkyl, C₆ to C₆₀ arylsulfonyl, heteroaryl having 5 to 60 nuclear atoms, and n may be selected from integers of 0 to 5.

As used herein, the term "aryl" refers to a monovalent substituent derived from an aromatic hydrocarbon having 6 to 40 carbon atoms, which is a single ring or is formed by combination of two or more rings. In addition, a form in which two or more rings are simply attached to each other (pendant) or condensed may also be included therein. Examples of such aryl include, but are not limited to, phenyl, naphthyl, phenanthryl, anthryl, and the like.

As used herein, the term "arylene" is an atomic group obtained by removing one hydrogen atom from an aromatic hydrocarbon, and also includes those having a condensed ring, those in which two or more independent benzene rings or condensed rings are bonded to each other directly or via a group such as vinylene. The arylene group may have any substituent described in the present invention, and the portion thereof except the substituent usually has about 6 to 60 carbon atoms. In addition, arylene including the substituent usually has about 6 to 100 carbon atoms in total. Examples of such an arylene group include, but are not limited to, a phenylene group, a naphthalenediyl group, an anthracene-diyl group, a biphenyl-diyl group, a terphenyl-diyl group, a condensed compound group, a fluorene-diyl group, a stilbene-diyl group, a distyrene diyl group, benzofluorene-diyl group, dibenzofluorene-diyl group, and the like.

As used herein, the term "alkyl" refers to a linear or branched saturated monovalent hydrocarbon radical, wherein the alkyl may be optionally substituted with one or more substituents described in the present invention. Examples of alkyl include, but are not limited to, methyl, ethyl, propyl (including all isomeric forms thereof), n-propyl, isopropyl, butyl (including all isomeric forms thereof), n-butyl, isobutyl, sec-butyl, t-butyl, pentyl (including all isomeric forms thereof), and hexyl (including all isomeric forms thereof).

As used herein, the term "heteroarylene" refers to a bivalent monocyclic aromatic group or a bivalent polycyclic aromatic group which contains at least one aromatic ring and the at least one aromatic ring contains, in the ring, one or more heteroatoms independently selected from O, S, and N. Each ring of heteroarylene group may contain one or two O atoms, one or two S atoms, and/or 1 to 4 N atoms, provided that the total number of heteroatoms in each ring is 4 or less and each ring must contain at least one carbon atom. Examples of heteroarylene include, but are not limited to, benzofuranylene, benzimidazolylene, benzoisoxazolylene, benzopyranylene, benzothiadiazolylene, benzothiazolylene, benzothienylene, benzotriazolylene, benzoxazolylene, furopyridylene, imidazopyridinylene, imidazothiazolylene, indolizinylene, indolylene, indazolylene, isobenzofuranylene, isobenzothienylene, isoindolylene, isoquinolinylene, isothiazolylene, naphthyridinylene, oxazolopyridinylene, phthalazinylene, pteridinylene, furinylene, pyridopyridylene, pyrrolopyridylene, quinolinylene, quinoxalinylene, quinazolinylene, thiadiazolopyrimidylene, and thienopyridylene. Examples of tricyclic heteroarylene group include, but are not limited to, acridinylene, benzindolylene, carbazolylene, dibenzofuranylene, perimidinylene, phenanthrolinylene, phenantridinylene, phenarsazinylene, phenazinylene, phenothiazinylene, phenoxazinylene and the like.

As used herein, the term "alkyl" refers to a linear or branched saturated monovalent hydrocarbon radical, wherein the alkyl may be optionally substituted with one or more substituents described in the present invention. Examples of alkyl include, but are not limited to, methyl, ethyl, propyl (including all isomeric forms thereof), n-propyl, isopropyl, butyl (including all isomeric forms thereof), n-butyl, isobutyl, sec-butyl, t-butyl, pentyl (including all isomeric forms thereof), and hexyl (including all isomeric forms thereof).

As used herein, the term "alkylsulfonyl group" includes a methylsulfonyl group, an ethylsulfonyl group, an n-propylsulfonyl group, an i-propylsulfonyl group, a t-butylsulfonyl group, and the like. The number of carbon atoms constituting the alkylsulfonyl group is preferably 1 to 10, but is not limited thereto.

As used herein, the term "alkenyl" refers to a linear or branched monovalent hydrocarbon radical that contains one or more carbon-carbon double bond(s), wherein the number of the carbon-carbon double bond(s) is 1 to 5, in an embodiment, and is one, in another embodiment. The alkenyl may be optionally substituted with one or more substituents described in the present invention. As understood by those skilled in the art, the term "alkenyl" includes radicals having a "cis" or "trans" structure or a mixture thereof, or alternatively a "Z" or "E" structure or a mixture thereof. Examples of alkenyl include, but are not limited to, ethenyl, propen-1-yl, propen-2-yl, allyl, butenyl, and 4-methylbutenyl.

As used herein, the term "aryloxy" is a monovalent substituent represented by RO-, wherein R means aryl having 5 to 40 carbon atoms. Examples of such aryloxy include, but are not limited to, phenyloxy, naphthyloxy, diphenyloxy, and the like.

As used herein, the term "heterocycloalkyl" refers to a monovalent monocyclic system having 3 to 20 ring atoms which contains 1 to 3 heteroatoms selected from N, O, P, or S, with the remaining ring atoms being C. One or more hydrogen atoms in the heterocycloalkyl group may be optionally substituted.

As used herein, the term "alkoxy" refers to a monovalent substituent represented by R'O-, wherein R' means an alkyl having 1 to 40 carbon atoms, and may include a linear, branched, or cyclic structure. Examples of alkyloxy include, but are not limited to, methoxy, ethoxy, n-propoxy, 1-propoxy, t-butoxy, n-butoxy, pentoxy, and the like.

As used herein, the term "arylamine" refers to amine substituted with aryl having 6 to 40 carbon atoms.

As used herein, the term "cycloalkyl" refers to a monovalent substituent derived from a monocyclic or polycyclic non-aromatic hydrocarbon having 3 to 40 carbon atoms. Examples of such cycloalkyl include, but are not limited to, cyclopropyl, cyclopentyl, cyclohexyl, norbornyl, adamantine, and the like.

As used herein, the term "halogen" refers to fluorine, chlorine, bromine, and/or iodine.

As used herein, the term "substituted alkyl", "substituted alkylene", "substituted heteroalkylene", "substituted alkenyl", "substituted alkenylene", "substituted heteroalkenylene", "substituted alkynyl", "substituted alkynylene", "substituted cycloalkyl", "substituted heterocycloalkyl", "substituted cycloalkylene", "substituted aryl", "substituted aryloxy", "substituted arylene", "substituted aralkyl", "substituted heteroaryl", "substituted heteroarylene", "substituted heterocyclic", or "substituted heterocyclylene" means that the substituted alkyl, the substituted alkenyl, the substituted alkenylene, the substituted cycloalkyl, the substituted heterocycloalkyl, the substituted cycloalkylene, the substituted aryl, the substituted aryloxy, the substituted arylene, the substituted aralkyl, the substituted heteroaryl, the substituted heteroarylene, the substituted heterocyclic, or the substituted heterocyclylene may be, each independently, further substituted with one or more substituents, for example, independently selected from the following:
In another embodiment of the present invention, there are provided compounds represented by the following formulas: 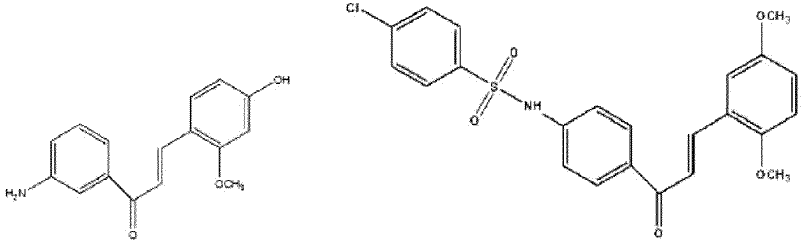 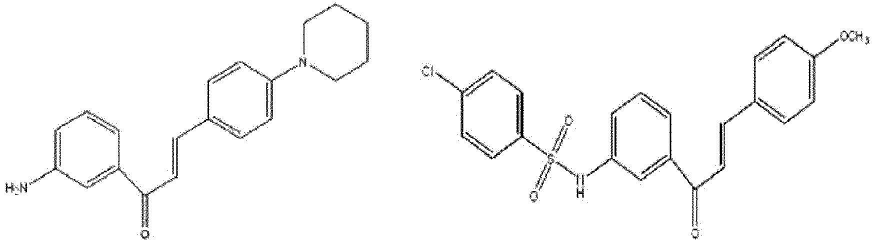 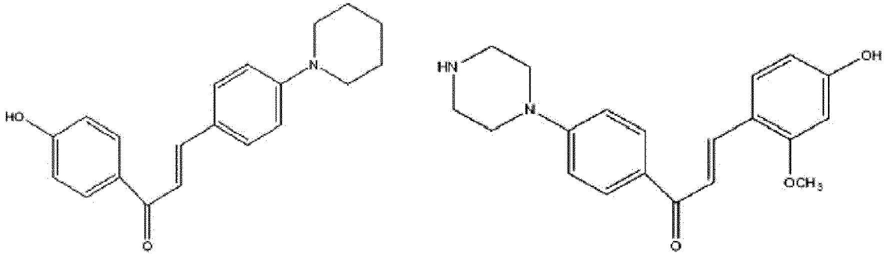 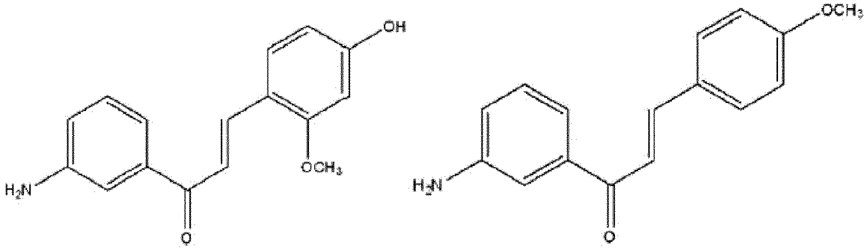 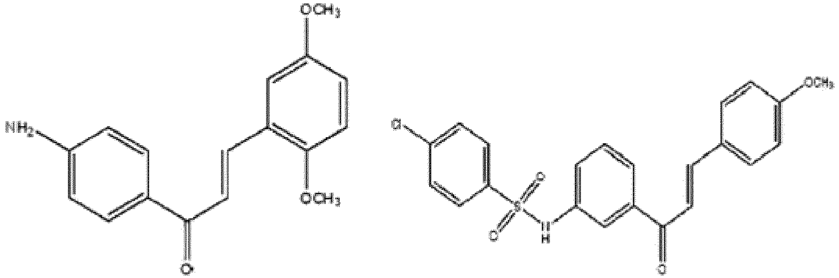 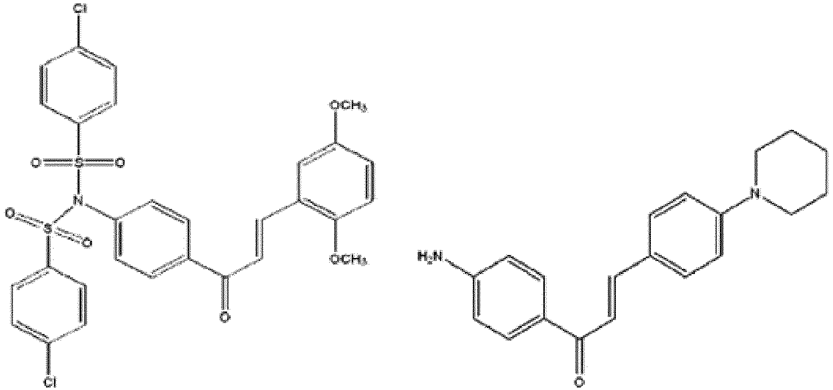 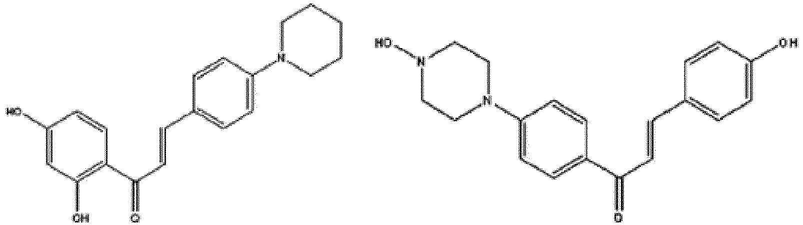 and

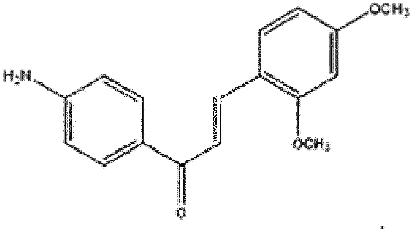

In yet another embodiment of the present invention, there is provided a method for preparing the compound according to the present invention, comprising the following steps. Specifically, the method may comprise 1) a step of mixing an acetophenone derivative and a benzaldehyde derivative with an organic solvent, and performing stirring, and 2) a step of extracting the reactant of the step 1) using an organic solvent.

As used herein, the term "organic solvent" may be any one selected from the group consisting of an alcohol-based solvent, a ketone-based solvent, a cellosolve-based solvent, a carboxylic acid-based solvent, a carbitol-based solvent, an acetate-based solvent, a lactate-based solvent, an amine-based solvent, an ether-based solvent, an aromatic hydrocarbon-based solvent, an aliphatic hydrocarbon-based solvent, and an amide-based solvent. Preferably the organic solvent may be ethyl acetate, but is not limited thereto.

As used herein, the term "acetophenone derivative" refers to a compound with an acetophenone structure as a mother body which has 1 or 2 substituents, and means, but is not limited to, any one selected from the group of 2-hydroxy-2-methyl-1-phenylpropane-1-one, 1-(4-isopropylphenyl)-2-hydroxy-2-methylpropan-1-one, 4-(2-hydroxyethoxy)-phenyl (2-hydroxy)propyl ketone, 1-hydroxycyclohexyl phenyl ketone, benzoin methyl ether, benzoin ethyl ether, benzoin isobutyl ether, benzoin butyl ether, 2,2-dimethoxy-2-phenyl acetophenone, 2-methyl-(4-methylthiophenyl)-2-morpholino-1-propan-1-one, 2-benzyl-2-dimethylamino-1-(4-morpholinophenyl)-1-butanone, 4-aminoacetophenone, or 3-aminoacetophenone. Preferably the acetophenone derivative may be 3-aminoacetophenone or 4-aminoacetophenone, but is not limited thereto.

As used herein, the term "benzaldehyde derivative" refers to a compound with a benzaldehyde structure as a mother body which has 1 or 2 substituents. The benzaldehyde derivative may be selected from, but is not limited to, any one of the compound 2,4-dimethoxybenzaldehyde, 2,5-dimethoxybenzaldehyde, 4-methoxybenzaldehyde, 2-methoxy-4-((tetrahydro-2H-pyran-2-yl)oxy)benzaldehyde, or 4-(piperidin-1-yl)benzaldehyde.

In the present invention, the method may further comprise, after the step 2), a step of adding a substituted alkylsulfonyl chloride-based compound and a base, and performing mixing and stirring.

However, in the present invention, examples of the substituted or unsubstituted alkylsulfonyl chloride-based compound include, but is not limited thereto, substituted methylsulfonyl chloride, substituted ethylsulfonyl chloride, substituted or unsubstituted n-propylsulfonyl chloride, substituted or unsubstituted i-propylsulfonyl chloride, substituted or unsubstituted t-butylsulfonyl chloride, and the like. The number of carbon atoms constituting alkylsulfonyl chloride is preferably 1 to 10, but is not limited thereto. Here, it is meant that the substituents may be, each independently, further substituted with one or more substituents, for example, independently selected from halogen atoms. More preferably, the alkylsulfonyl chloride may be 4-chlorobenzenesulfonyl chloride, but is not limited thereto.

In still yet another embodiment of the present invention, there is provided a pharmaceutical composition for preventing or treating degenerative neurological diseases, comprising the compound according to the present invention as an active ingredient.

In the present invention, the degenerative neurological disease refers to a disease occurring in the brain among degenerative diseases which develop with age. Specifically, in consideration of the main symptom and the affected brain part, the degenerative neurological disease may be one or more selected from the group consisting of stroke, palsy, dementia, Alzheimer's disease, Huntington's disease, amyotrophic lateral sclerosis (ALS), Pick's disease, and Creutzfeldt-Jakob disease, and may be preferably, but is not limited to, Alzheimer's disease.

In the present invention, according to the amyloid hypothesis, the degenerative neurological disease, in particular, Alzheimer's disease may develop as brain neuron damage is caused by stress resulting from over-expression of beta-amyloid by amyloid precursor protein (APP), presensilin 1/2 (PS1/2), or apolipoprotein E (Apo E) due to genetic or environmental factors, or from decreased clearance of beta-amyloid with neuronal toxicity.

In the present invention, the pharmaceutical composition may be characterized by being in the form of capsules, tablets, granules, injections, ointments, powders, or beverages, and the pharmaceutical composition may be characterized by being targeted for humans.

In the present invention, the pharmaceutical composition may be formulated in the form of, but is not limited thereto, oral formulations such as powders, granules, capsules, tablets, and aqueous suspensions, formulations for external use, suppositories, and sterile injectable solutions, according to conventional methods, respectively. The pharmaceutical composition of the present invention may comprise a pharmaceutically acceptable carrier. For oral administration, as the pharmaceutically acceptable carrier, binders, glidants, disintegrants, excipients, solubilizing agents, dispersing agents, stabilizers, suspending agents, pigments, fragrances, and the like may be used. For injections, as the pharmaceutically acceptable carrier, buffers, preservatives, pain-relieving agents, solubilizing agents, isotonic agents, stabilizers, and the like may be mixed and used. For local administration, as the pharmaceutically acceptable carrier, bases, excipients, lubricants, preservatives, and the like may be used. The formulations of the pharmaceutical composition of the present invention may be prepared in various ways by being mixed with the pharmaceutically acceptable carrier as described above. For example, for oral administration, the pharmaceutical composition may be prepared in the form of tablets, troches, capsules, elixirs, suspensions, syrups, wafers, or the like. For injections, the pharmaceutical composition may be prepared in the form of unit dosage ampoules or multiple dosage forms. The pharmaceutical composition may be formulated into others, solutions, suspensions, tablets, capsules, sustained-release preparations, or the like.

Meanwhile, examples of carriers, excipients, and diluents, which are suitable for formulation, include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. In addition, fillers, anti-coagulants, lubricants, wetting agents, fragrances, emulsifiers, preservatives, and the like may further be included.

Routes of administration for the pharmaceutical composition according to the present invention include, but are not limited to, oral, intravenous, intramuscular, intraarterial, intramedullary, intrathecal, intracardiac, transdermal, subcutaneous, intraperitoneal, intranasal, intestinal, topical, sublingual, or intrarectal route. Oral or parenteral administration is preferred.

As used herein, the term "parenteral" is meant to include subcutaneous, intradermal, intravenous, intramuscular, intraarticular, intrabursal, intrasternal, intrathecal, intralesional, and intracranial injection or infusion techniques. The pharmaceutical composition of the present invention may also be administered in the form of suppositories for rectal administration.

The pharmaceutical composition of the present invention may vary depending on various factors including activity of a particular compound used, the patient's age, body weight, general health, sex, diet, time of administration, route of administration, excretion rate, drug combination, and severity of a particular disease to be prevented or treated. A dose of the pharmaceutical composition may vary depending on the patient's condition, body weight, severity of disease, drug form, route of administration, and period of administration, and may be appropriately selected by those skilled in the art. The pharmaceutical composition may be administered at a dose of 0.0001 to 50 mg/kg/day or 0.001 to 50 mg/kg/day. The dose may be administered once a day or may be divided into several times a day. The dose does not limit the scope of the present invention in any way. The pharmaceutical composition according to the present invention may be formulated into pills, sugar-coated tablets, capsules, liquids, gels, syrups, slurries, or suspensions.

In other embodiments of the present invention, the compound according to the present invention may induce AMP-activated protein kinase (AMPK) enzyme activation.

As used herein, the term "AMP-activated protein kinase (AMPK) enzyme" refers to an enzyme which acts as a sensor for maintaining energy homeostasis in a cell and which promotes a process of consuming ATP by being activated in a case where energy in a cell decreases due to metabolic stress or exercise, that is, in a case where ATP is depleted and an AMP/ATP ratio increases (Nat Rev Mol Cell Biol 8: 774-785, 2007). For the purpose of the present invention, the novel compound according to the present invention itself is not directly involved in the enzymatic reaction, and acts to activate an inactive enzyme. The compound binds to an activation site on the AMP-activated protein kinase enzyme, to increase activity of the enzyme, so that the compound can be used to prevent or treat symptoms of degenerative neurological diseases. More specifically, the compound can increase autophagy of the AMP-activated protein kinase enzyme, and thus increase an inhibitory effect on accumulation of beta-amyloid, thereby acting to prevent or treat symptoms of degenerative neurological diseases. However, the action of the compound is not limited thereto.

### Advantageous Effects of Invention

The novel compound according to the present invention, which is obtained by means of carrying out chemical modification in a mother structure showing excellent AMP-activated protein kinase (AMPK) activation efficacy and in silico binding capacity, enables effective prevention or treatment of degenerative neurological disorders by means of effectively inducing AMP-activated protein kinase enzyme activation.

### Brief Description of Drawings

FIG. 1 illustrates an in silico schematic diagram according to an embodiment of the present invention.
FIG. 2 illustrates results obtained by performing virtual selection for a preparation example according to an embodiment of the present invention.
FIG. 3 illustrates results obtained by performing a kinase assay according to an embodiment of the present invention.
FIG. 4 illustrates a circular pool for Morris water maze test according to an embodiment of the present invention.
FIG. 5 illustrates results obtained by performing Morris water maze test according to an embodiment of the present invention.
FIG. 6 illustrates results obtained by performing Morris water maze test according to an embodiment of the present invention.
FIG. 7 illustrates results obtained by performing Morris water maze test according to an embodiment of the present invention.
FIG. 8 illustrates results obtained by performing a probe test according to an embodiment of the present invention.
FIG. 9 illustrates results obtained by performing a probe test according to an embodiment of the present invention.
FIG. 10 illustrates results obtained by performing a passive avoidance test according to an embodiment of the present invention.
FIG. 11 illustrates results obtained by performing a rotarod test according to an embodiment of the present invention.
FIG. 12 illustrates results obtained by performing a vertical pole test according to an embodiment of the present invention.
FIG. 13 illustrates results obtained by measuring activity of acetylcholinesterase (AchE) according to an embodiment of the present invention.
FIG. 14 illustrates results obtained by measuring acetylcholine (Ach) according to an embodiment of the present invention.
FIG. 15 illustrates results obtained by performing TUNEL assay according to an embodiment of the present invention.
FIG. 16 illustrates results obtained by performing TUNEL assay according to an embodiment of the present invention.
FIG. 17 illustrates analysis of degree of membrane permeability for Preparation Example 6.
FIG. 18 illustrates results obtained by performing half-life analysis for Preparation Example 6.

### Detailed Description of Invention

The present invention provides a novel compound, (E)-4-chloro-N-(4-(3-(2,5-dimethoxyphenyl)acryloyl)phenyl)benzenesulfonamide, and provides a pharmaceutical composition for preventing or treating degenerative neurological diseases, comprising the compound as an active ingredient.

Hereinafter, the present invention will be described in more detail by way of examples. These examples are only for describing the present invention in more detail, and it will be apparent to those skilled in the art that according to the gist of the present invention, the scope of the present invention is not limited by these examples.

### [Experimental Example 1] Selection of drug candidates with AMP-activated protein kinase activation efficacy for treatment of degenerative neurological diseases

In order to select candidates for the treatment of degenerative neurological diseases, drug candidates capable of binding to AMP-activated protein kinase were selected.

Selection of the candidates was made by identifying, through in silico and in vitro screening methods, candidates capable of binding to a regulatory site on the AMP-activated protein kinase enzyme, from a chemical library.

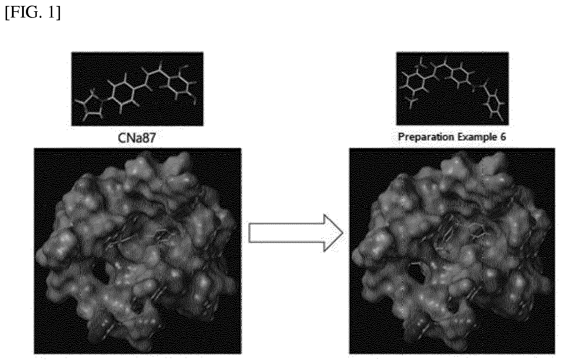

As illustrated in FIG. 1, CNa87, which is the form that can best bind to the AMP-activated protein kinase enzyme, was finally selected.

Using CNa87 as a mother body, Preparation Examples 1 to 15, which are candidates obtained by modifying CNa87 with a group that is bulkier than a hydroxyl group so as to have further enhanced binding ability to the AMP-activated protein kinase enzyme, were synthesized by the following methods.

### [Preparation Examples 1 to 15] Synthesis of candidates

Preparation Examples 1 to 15 in Table 1 below were prepared by the following Method 1 or Method 2.

**[Table 1]**

| Preparation example | Compound name |
|---|---|
| Preparation Example 1 | (E)-1-(4-Aminophenyl)-3-(2,4-dimethoxyphenyl)prop-2-en-1-one |
| Preparation Example 2 | (E)-1-(4-Aminophenyl)-3-(2,5-dimethoxyphenyl)prop-2-en-1-one |
| Preparation Example 3 | (E)-1-(3-Aminophenyl)-3-(4-methoxyphenyl)prop-2-en-1-one |
| Preparation Example 4 | (E)-1-(3-Aminophenyl)-3-(4-hydroxy-2-methoxyphenyl)prop-2-en-1-one |
| Preparation Example 5 | (E)-1-(3-Aminophenyl)-3-(2,5-dimethoxyphenyl)prop-2-en-1-one |
| Preparation Example 6 | (E)-4-Chloro-N-(4-(3-(2,5-dimethoxyphenyl)acryloyl)phenyl)benzenesulfonamide |
| Preparation Example 7 | (E)-4-Chloro-N-(3-(3-(4-methoxyphenyl)acryloyl)phenyl)benzenesulfonamide |
| Preparation Example 8 | (E)-4-Chloro-N-(3-(3-(2,5-dimethoxyphenyl)acryloyl)phenyl)benzenesulfonamide |
| Preparation Example 9 | (E)-4-Chloro-N-((4-chforophenyl)sulfonyl)-N-(3-(3-(2,5-dimethoxyphenyl)acryloyl)phenyl)benzenesulfonamide |
| Preparation Example 10 | (E)-1-(4-Aminophenyl)-3-(4-(piperidin-1-yl)phenyl)prop-2-en-1-one |
| Preparation Example 11 | (E)-1-(3-Aminophenyl)-3-(4-(piperidin-1-yl)phenyl)prop-2-en-1-one |
| Preparation Example 12 | (E)-1-(4-Hydroxyphenyl)-3-(4-(piperidin-1-yl)phenyl)prop-2-en-1-one |
| Preparation Example 13 | (E)-1-(2,4-Dihydroxyphenyl)-3-(4-(piperidin-1-yl)phenyl)prop-2-en-1-one |
| Preparation Example 14 | (E)-3-(4-Hydroxy-2-methoxyphenyl)-1-(4-(piperazin-1-yl)phenyl)prop-2-en-1-one |
| Preparation Example 15 | (E)-3-(4-Hydroxyphenyl)-1-(4-(4-methylpiperazin-1-yl)phenyl)prop-2-en-1-one |

### [Method 1]

An acetophenone derivative (1 equivalent), a benzaldehyde derivative (1 equivalent), and NaOH (1 equivalent) were added to an ethanol solvent and stirring was performed at room temperature. Water was added to the reaction mixture and extraction with ethyl acetate was performed. The organic solvent layer was collected and washed once again with water. Anhydrous MgSO₄ was added thereto and dehydration was performed. Then, the solvent was distilled off under reduced pressure, and the remaining residue was purified by silica gel chromatography, to prepare the compound.

### [Method 2]

An acetophenone derivative (1 equivalent), 4-((tetrahydro-2H-pyran-2-yl)oxy))benzaldehyde derivative (1 equivalent), and NaOH (1 equivalent) were added to an ethanol solvent and stirring was performed at room temperature. 4M HCl was added to the reaction mixture and stirred for 20 more minutes. Then, water was added thereto and extraction with ethyl acetate was performed. The organic solvent layer was collected and washed once again with water. Anhydrous MgSO₄ was added thereto and dehydration was performed. Then, the solvent was distilled off under reduced pressure, and the remaining residue was purified by silica gel chromatography, to prepare the compound.

### [Preparation Example 11 (E)-1-(4-Aminophenyl)-3-(2,4-dimethoxyphenyl)prop-2-en-1-one)

According to the above Method 1, 4-aminoacetophenone (0.30 g, 2.22 mmol), 2,4-dimethoxybenzaldehyde (0.37 g, 2.22 mmol), and NaOH (0.09 g, 2.22 mmol) were used and purification by silica gel chromatography (developing solvent: ethyl acetate/n-hexane = 1:2 1:1) was performed, to obtain yellow Preparation Example 1 (0.15 g, 23.0%). Rf 0.33 (ethyl acetate/n-hexane = 1:1); ¹H-NMR (400MHz, CDCl₃) δ 3.85 (s, 3H), 3.89 (s, 3H), 6.47 (d, J = 2.0 Hz, 1H), 6.52(dd, J = 8.4, 2.4 Hz, 1H), 6.69 (d, J = 8.4 Hz, 2H), 7.55 (d, J = 15.6 Hz, 1H), 7.56 (d, J = 8.8 Hz, 1H), 7.92 (d, J = 8.4 Hz, 2H), 8.02 (d, J = 15.6 Hz, 1H); ¹³C-NMR (100MHz, CDCl₃) 55.6, 55.7, 98.7, 105.5, 114.1, 117.8, 120.7, 129.4,130.8, 131.1, 139.0, 150.9, 160.4, 162.8, 189.1 ppm.

### [Preparation Example 21 (E)-1-(4-Aminophenyl)-3-(2,5-dimethoxyphenyl)prop-2-en-1-one)

According to the above Method 1, 4-aminoacetophenone (0.50 g, 3.70 mmol), 2,5-dimethoxybenzaldehyde (0.62 g, 3.70 mmol), and NaOH (0.15 g, 3.70 mmol) were purified by silica gel chromatography (developing solvent: ethyl acetate/n-hexane = 1:2 1:1), to obtain yellow Preparation Example 2 (0.66 g, 62.5%). Rf 0.36 (ethyl acetate/n-hexane = 1:1); ¹H-NMR (400 MHz, CDCl₃) δ 3.81 (s, 3H), 3.85 (s, 3H), 4.19 (br s, 2H), 6.69 (d, J = 8.8 Hz, 2H), 6.86 (d, J = 8.8 Hz, 1H), 6.91 (dd, J = 8.8, 2.8 Hz, 1H), 7.16 (d, J = 2.8 Hz, 1H), 7.58 (d, J = 15.6 Hz, 1H), 7.92 (d, J =8.8 Hz, 2H), 8.04 (d, J = 15.6 Hz, 1H); ¹³C-NMR (100 MHz, CDCl₃) 56.0, 56.3, 112.7, 113.9, 114.1, 116.8, 123.3, 125.2, 128.9, 131.3, 138.6, 151.2, 153.4, 153.7, 188.8 ppm.

### [Preparation Example 3] (E)-1-(3-Aminophenyl)-3-(4-methoxyphenyl)prop-2-en-1-one)

According to the above Method 1, 3-aminoacetophenone (1.00 g, 7.40 mmol), 4-methoxybenzaldehyde (1.00 g, 7.40 mmol), and NaOH (0.30 g, 7.40 mmol) were used and purification by silica gel chromatography (developing solvent: ethyl acetate/n-hexane = 1:2 1:1) was performed, to obtain orange Preparation Example 3 (0.83 g, 62.5%). R_{f} 0.40 (ethyl acetate/n-hexane = 1:1); ¹H-NMR (400 MHz, CDCl₃) δ 3.83 (br s, 2H), 3.85(s, 3H), 6.88 (ddd, J = 8.0, 2.4, 0.8Hz, 1H), 6.93 (d, J = 8.8 Hz, 2H), 7.27(dd, J = 8.0, 7.6 Hz, 1H), 7.31 (dd, J = 2.0, 1.6 Hz, 1H), 7.36 (d, J = 15.6 Hz, 1H), 7.38 (ddd, J = 7.6, 1.6, 0.8 Hz, 1H), 7.59 (d, J = 8.8 Hz, 2H), 7.76 (d, J = 15.6 Hz, 1H); ¹³C-NMR(100 MHz, CDCl₃) 56.0, 56.3, 112.7, 113.9, 114.1, 116.8, 123.3,125.2, 128.9, 131.3, 138.6, 151.2, 153.4, 153.7, 188.8 ppm.

### [Preparation Example 4] (E)-1-(3-Aminophenyl)-3-(4-hydroxy-2-methoxyphenyl)prop-2-en-1-one)

According to the above Method 2, 3-aminoacetophenone (0.40 g, 2.96 mmol), 2-methoxy-4-((tetrahydro-2H-pyran-2-yl)oxy)benzaldehyde (0.70 g, 2.96 mmol), and NaOH (0.12 g, 2.96 mmol) were used and purification by silica gel chromatography (developing solvent: ethyl acetate/n-hexane = 1:1) was performed, to obtain orange Preparation Example 4 (0.28 g, 35.1%). R_{f} 0.25 (ethyl acetate/n-hexane = 1:1); ¹H-NMR (400 MHz, CDCl₃) δ 3.71 (s, 3H), 6.30 (d, J = 2.4 Hz, 1H), 6.34 (dd, J = 8.4, 2.0 Hz, 1H), 6.72 (ddd, J= 8.0, 2.4, 0.8 Hz, 1H), 7.08 (dd, J = 8.4, 8.0 Hz, 1H), 7.13 (d, J =2.4 Hz, 1H), 7.16 (ddd, J = 7.6, 7.6, 0.8 Hz, 1H), 7.29 (d, J = 15.6Hz, 1H), 7.32 (d, J = 8.4 Hz, 1H), 7.85 (d, J = 15.6 Hz, 1H), 9.35(br s, 1H); ¹³C-NMR (100 MHz, CDCl₃) 55.3, 99.1, 108.2, 114.1,115.3, 118.1, 118.7,119.3, 129.1, 130.6, 139.8, 140.3, 147.0, 60.5, 161.4, 191.1 ppm.

### [Preparation Example 5] (E)-1-(3-Aminophenyl)-3-(2,5-dimethoxyphenyl)prop-2-en-1-one)

According to the above Method 1, 4-aminoacetophenone (0.50 g, 3.70 mmol), 2,5-dimethoxybenzaldehyde (0.62 g, 3.70 mmol), and NaOH (0.15 g, 3.70 mmol) were used and purification by silica gel chromatography (developing solvent: ethyl acetate/n-hexane = 1:3) was performed, to obtain yellow Preparation Example 5 (0.27 g, 25.4%). Rf 0.62 (ethyl acetate/n-hexane = 1:1); ¹H-NMR (400 MHz, CDCl₃) δ 3.81 (s, 3H), 3.86 (s,3H), 6.87 (d, J = 8.8 Hz, 1H), 6.88(ddd, J = 8.4, 2.0, 0.8 Hz, 1H), 6.94(dd, J = 8.8, 2.8 Hz, 1H), 7.16 (d, J = 2.8 Hz, 1H), 7.27 (dd, J = 8.0, 8.0 Hz, 1H), 7.31 (dd, J = 2.8, 2.8 Hz, 1H), 7.38 (ddd, J = 8.0, 2.0, 1.6 Hz, 1H), 7.53 (d, J = 15.6 Hz, 1H), 8.06 (d, J = 15.6 Hz, 1H); ¹³C-NMR(100 MHz, CDCl₃) 56.1, 56.3, 112.7, 113.9, 114.7, 117.4, 119.1,119.4, 123.6, 124.8, 129.6, 139.8, 140.0, 147.0, 153.5, 153.7, 191.4 ppm.

### [Preparation Example 6] (E)-4-Chloro-N-(4-(3-(2,5-dimethoxyphenyl)acryloyl)phenyl)benzenesulfonamide

4-Chlorobenzenesulfonyl chloride (0.75 g, 3.54 mmol) was added to a CH₂Cl₂ solution in which the Preparation Example 2 (0.67 g, 2.36 mmol) and triethylamine (TEA, 0.26 g, 2.60 mmol) are dissolved, and stirring was performed at room temperature for 24 hours. Water was added to the reaction mixture, and extraction with ethyl acetate was performed. The organic solvent layer was collected and washed with saturated NaHCO₃. Anhydrous MgSO₄ was added thereto and dehydration was performed. Then, the solvent was distilled off under reduced pressure, and the remaining residue was purified by silica gel chromatography (developing solvent: ethyl acetate/n-hexane = 1:2), to obtain yellow Preparation Example 6 (0.55 g, 50.9%). R_{f} 0.18 (ethyl acetate/n-hexane = 1:1); ¹H-NMR (400 MHz, DMSO-d6) δ 3.74 (s, 3H), 3.80 (s, 3H), 6.84 (d, J = 8.8 Hz, 1H), 6.88 (dd, J= 8.8, 2.4 Hz, 1H), 7.16 (d, J = 2.4Hz, 1H), 7.20 (d, J = 8.4 Hz, 2H), 7.40 (d, J = 8.4 Hz, 2H), 7.54 (d, J = 15.6 Hz, 1H), 7.74 (d, J = 8.4 Hz, 2H), 7.85 (d, J = 8.4 Hz, 2H), 7.91 (d, J = 15.6 Hz, 1H), 10.58 (s, 1H); ¹³C-NMR(100 MHz, DMSO-d6) 55.2, 55.6, 112.1, 112.7, 117.0, 118.0, 121.6,123.5, 128.0, 128.1, 128.7, 129.4, 132.9, 137.8, 138.3, 141.5, 152.5, 152.9,187.9 ppm.

### [Preparation Example 7] (E)-4-Chloro-N-(3-(3-(4-methoxyphenyl)acryloyl)phenyl)benzenesulfonamide

N-(3-Acetylphenyl)-4-chlorobenzenesulfonamide (0.10 g, 0.32 mmol), 4-methoxybenzaldehyde (0.04 g, 0.32 mmol), and NaOH (0.03 g, 0.80 mmol) was added to an ethanol solvent and stirring was performed at room temperature for 72 hours. A dilute hydrochloric acid aqueous solution was added to the reaction mixture, and extraction with ethyl acetate was performed. The organic solvent layer was collected and washed with water. Anhydrous MgSO₄ was added thereto and dehydration was performed. Then, the solvent was distilled off under reduced pressure. Subsequently, the remaining residue was purified by silica gel chromatography (developing solvent: ethyl acetate/n-hexane = 1:3 2:1), to obtain yellow Preparation Example 7 (0.01 g, 6.5%). ¹H-NMR (400 MHz, CDCl₃) δ 3.84 (s, 3H), 6.92 (d, J = 8.8 Hz, 2H), 7.28 (d, J = 15.6 Hz, 1H), 7.33 (dd, J = 8.0, 7.6 Hz, 1H), 7.36 (d, J = 8.8 Hz, 2H), 7.41 (ddd, J = 8.0, 2.0, 1.2 Hz, 1H), 7.57 (d, J = 8.8 Hz, 2H), 7.68 (ddd, J = 7.6, 1.6, 1.2 Hz, 1H), 7.71 - 7.78(m, 4H), 9.25 (s, 1H); ¹³C-NMR (100 MHz, CDCl₃) 55.6,114.6, 119.5, 121.1, 124.9, 125.0, 127.6, 128.8, 129.4, 129.7, 130.5, 137.

### [Preparation Example 8] (E)-4-Chloro-N-(3-(3-(2,5-dimethoxyphenyl)acryloyl)phenyl)benzenesulfonamide

4-Chlorobenzenesulfonyl chloride (0.09 g, 0.43 mmol) was added to a CH₂Cl₂ solution in which the Preparation Example 5 (0.12 g, 0.43 mmol) and triethylamine (0.03 g, 2.60 mmol) are dissolved, and stirring was performed at room temperature for 24 hours. Water was added to the reaction mixture, and extraction with ethyl acetate was performed. The organic solvent layer was collected and washed with saturated NaHCO₃. Anhydrous MgSO₄ was added thereto and dehydration was performed. Then, the solvent was distilled off under reduced pressure, and the remaining residue was purified by silica gel chromatography (developing solvent: ethyl acetate/n-hexane = 1:3), to obtain yellow Preparation Example 8 (0.08 g, 37.3%). Rf 0.33 (ethyl acetate/n-hexane = 1:1); ¹H-NMR (400 MHz, CDCl₃) δ 3.82 (s, 3H), 3.88 (s, 3H), 6.88 (d, J = 8.8 Hz, 1H), 6.96 (dd, J= 8.4, 2.8 Hz, 1H), 7.15 (d, J = 2.8Hz, 1H), 7.40 (d, J = 8.8 Hz, 2H),7.41 -7.43 (m, 2H), 7.55 (d, J = 16.0Hz, 1H), 7.73 (d, J = 8.8 Hz, 2H),7.71 - 7.72 (m, 1H), 7.76 - 7.79 (m, 1H), 8.08 (d, J = 16.0 Hz, 1H); ¹³C-NMR (100 MHz, CDCl₃)56.1, 56.3, 112.7, 114.5, 117.9, 121.6, 122.8, 124.4, 125.5, 125.8, 128.9,129.7, 130.0, 137.1,137.7, 139.9, 140.0, 141.6, 153.7, 153.8, 190.3 ppm.

### [Preparation Example 9] (E)-4-Chloro-N-((4-chlorophenyl)sulfonyl)-N-(3-(3-(2,5-dimethoxyphenyl)acryloyl)phenyl)benzenesulfonamide

4-Chlorobenzenesulfonyl chloride (0.25 g, 1.17 mmol) was added to a CH₂Cl₂ solution in which the Preparation Example 5 (0.22 g, 0.78 mmol) and triethylamine (0.22 g, 2.12 mmol) are dissolved, and stirring was performed at room temperature for 24 hours. Water was added to the reaction mixture, and extraction with ethyl acetate was performed. The organic solvent layer was collected and washed with saturated NaHCO₃. Anhydrous MgSO₄ was added thereto and dehydration was performed. Then, the solvent was distilled off under reduced pressure, and the remaining residue was purified by silica gel chromatography (developing solvent: ethyl acetate/n-hexane = 1:4), to obtain yellow Preparation Example 9 (0.20 g, 39.8%). R_{f} 0.77 (ethyl acetate/n-hexane = 1:1); ¹H-NMR (400 MHz,CDCl3) δ 3.83 (s, 3H), 3.86 (s, 3H), 6.89 (d, J = 9.2 Hz, 1H), 6.97 (dd, J= 9.2, 3.2 Hz, 1H), 7.13 (d, J = 2.8Hz, 1H), 7.19 (dd, J = 8.4, 2.0 Hz,1H), 7.42 (d, J = 16.0 Hz, 1H), 7.52(d, J = 8.4 Hz, 1H), 7.54 (d, J = 8.8 Hz, 4H), 7.66 (dd, J = 1.6, 1.6 Hz, 1H), 7.89 (d, J = 8.8 Hz, 4H), 8.05 (d, J = 16.0 Hz, 1H), 8.09 (d, J = 8.0 Hz, 1H),; ¹³C-NMR(100 MHz, CDCl₃) 56.1, 56.3, 112.7, 114.3, 117.9, 122.9, 124.3,129.8, 129.9, 130.3, 130.6, 131.6, 134.6, 135.2, 137.8, 140.2, 141.4, 141.8,153.7, 153.8, 189.9 ppm.

### [Preparation Example 10] (E)-1-(4-Aminophenyl)-3-(4-(piperidin-1-yl)phenyl)prop-2-en-1-one)

According to the above Method 1, 4-aminoacetophenone (0.40 g, 2.96 mmol), 4-(piperidin-1-yl)benzaldehyde (0.56 g, 2.96 mmol), and NaOH (0.12 g, 2.96 mmol) were used and purification by silica gel chromatography (developing solvent: MeOH:CHCl3 = 1:19) was performed, to obtain yellow Preparation Example 10 (0.28 g, 30.9%). Rf 00.43 (ethyl acetate/n-hexane = 1:1); ¹H-NMR(400 MHz, CDCl₃) δ 1.62 - 1.71 (m, 6H), 3.29 (t, J = 5.2 Hz, 4H), 4.10 (br s, 2H), 6.69(d, J = 8.8 Hz, 2H), 6.89 (d, J = 8.8 Hz, 2H), 7.37 (d, J = 15.6 Hz, 1H), 7.53 (d, J = 8.8 Hz, 2H), 7.75 (d, J = 15.6 Hz, 1H), 7.95 (d, J = 8.8 Hz, 2H).

### [Preparation Example 11] (E)-1-(3-Aminophenyl)-3-(4-(piperidin-1-yl)phenyl)prop-2-en-1-one)

According to the above Method 1, 3-aminoacetophenone (0.40 g, 2.96 mmol), 4-(piperidin-1-yl)benzaldehyde (0.56 g, 2.96 mmol), and NaOH (0.12 g, 2.96 mmol) were used and purification by silica gel chromatography (developing solvent: MeOH:CHCl₃ = 1:19) was performed, to obtain yellow Preparation Example 11 (0.45 g, 49.6%). Rf0.47 (ethyl acetate/n-hexane = 1:1); ¹H-NMR(400 MHz, CDCl₃) δ 1.61 - 1.72 (m, 6H), 3.31 (t, J = 5.6 Hz, 4H), 3.80 (br s, 2H), 6.86(ddd, J = 8.0, 2.4, 0.8 Hz, 1H), 6.89(d, J = 8.8 Hz, 2H), 7.28 (dd, J = 8.0, 8.0 Hz, 1H), 7.30 (d, J = 15.6 Hz, 1H), 7.31 (dd, J = 2.0, 2.0 Hz, 1H), 7.37 (ddd, J = 8.8, 1.2, 1.2 Hz, 1H), 7.53 (d, J = 8.8 Hz, 2H), 7.75 (d, J = 15.6 Hz, 1H); ¹³C-NMR(100 MHz, CDCl₃) 24.6, 25.7, 49.3, 114.7, 115.0, 118.4, 119.0,119.1, 124.7, 129.5, 130.4, 140.3, 145.4, 146.9, 153.4, 191.1 ppm.

### [Preparation Example 12](E)-1-(4-Hydroxyphenyl)-3-(4-(piperidin-1-yl)henyl)prop-2-en-1-one)

According to the above Method 2, 1-(4-((tetrahydro-2H-pyran-2-yl)oxy)phenyl)ethan-1-one (0.50g, 2.27 mmol), 4-(piperidin-1-yl)benzaldehyde (0.43 g, 2.27 mmol), and NaOH (0.09 g, 2.27 mmol) were used and purification by silica gel chromatography (developing solvent: ethyl acetate/n-hexane = 1:3 1:1) was performed, to obtain orange Preparation Example 12 (0.24 g, 33.7%). Rf 0.17 (ethyl acetate/n-hexane = 1:3); ¹H-NMR(400 MHz, CDCl₃) δ 1.58 - 1.65 (m, 6H), 3.25 (t, J = 5.6 Hz, 4H), 6.84 (d, J = 8.8 Hz, 2H), 6.87 (d, J = 8.8 Hz, 2H), 7.32 (d, J = 15.6 Hz, 1H), 7.48 (d, J = 8.8 Hz, 2H), 7.68 (d, J = 15.6 Hz, 1H), 7.90 (d, J = 8.8 Hz, 2H), 9.34 (s, 1H); ¹³C-NMR(100 MHz, CDCl₃) 24.4, 25.5, 49.2,114.9, 115.6, 117.8, 124.8,130.1, 130.6, 130.9, 144.2, 153.1, 161.7, 189.0 ppm.

### [Preparation Example 13] (E)-1-(2,4-Dihydroxyphenyl)-3-(4-(piperidin-1-yl)phenyl)prop-2-en-1-one)

According to the above Method 2, 1-(4-((tetrahydro-2H-pyran-2-yl)oxy)phenyl)ethan-1-one (0.50 g, 2.12 mmol), 4-(piperidin-1-yl)benzaldehyde (0.40 g, 2.12 mmol), and Ba(OH)₂ 8H₂O (0.73 g, 2.33 mmol) were used and purification by silica gel chromatography (developing solvent: ethyl acetate/n-hexane = 1:1) was performed, to obtain orange Preparation Example 13 (0.12 g, 16.8%). Rf 0.66 (ethyl acetate/n-hexane = 1:1); ¹H-NMR (400 MHz, CDCl₃) δ 1.58 - 1.65 (m, 6H), 3.27 (t, J = 5.6 Hz, 4H), 6.37 - 6.40 (m, 2H), 6.84 (d, J = 8.8 Hz, 2H), 7.34 (d, J= 15.2 Hz, 1H), 7.49 (d, J = 8.8 Hz, 2H), 7.73 (d, J = 8.4 Hz, 1H), 7.76(d, J = 15.2 Hz, 1H), 9.64 (s, 1H), 13.6 (s, 1H).

### [Preparation Example 14] (E)-3-(4-Hydroxy-2-methoxyphenyl)-1-(4-(piperazin-1-yl)phenyl)prop-2-en-1-one)

According to the above Method 2, 1-(4-((tetrahydro-2H-pyran-2-yl)oxy)phenyl)ethan-1-one (0.50 g, 2.45 mmol), 2-methoxy-4-((tetrahydro-2H-pyran-2-yl)oxy)benzaldehyde (0.58 g, 2.45 mmol), and NaOH (0.20 g, 4.90 mmol) were used, and the solid obtained after removal of the solvent was treated with a mixed solvent of ethyl acetate/n-hexane. The resulting solid was filtered and dried in vacuo, to obtain orange Preparation Example 14 (0.28 g, 33.8%). ¹H-NMR(400 MHz, DMSO-d6) δ 2.89 (dd, J= 7.2, 3.2 Hz, 4H), 3.26 (dd, J = 7.2,4.0 Hz, 4H), 3.82 (s, 3H), 6.40 (dd, J =8.8, 2.0 Hz, 1H), 6.41 (d, J = 1.6Hz, 1H), 6.89 (d, J = 8.8 Hz, 2H),7.51 (d, J = 15.6 Hz, 1H), 7.55 (d, J = 8.8 Hz, 1H), 7.87 (d, J = 15.6 Hz, 1H), 7.90 (d, J = 8.8 Hz, 2H),; ¹³C-NMR(100 MHz, DMSO-d6) 45.1, 47.5, 55.1, 98.7, 107.9, 112.9, 114.7,118.0, 127.7, 129.7, 129.8, 137.7, 153.7, 159.7, 161.2, 186.8 ppm.

### [Preparation Example 15] (E)-3-(4-Hydroxyphenyl)-1-(4-(4-methylpiperazin-1-yl)phenyl)prop-2-en-1-one

According to the above Method 2, 1-(4-((tetrahydro-2H-pyran-2-yl)oxy)phenyl)ethan-1-one (0.50 g, 2.29 mmol), 4-((tetrahydro-2H-pyran-2-yl)oxy)benzaldehyde (0.47 g, 2.29 mmol), and NaOH (0.09 g, 2.29 mmol) were used, and the solid obtained after removal of the solvent was treated with a mixed solvent of ethyl acetate/n-hexane. The resulting solid was filtered and dried in vacuo, to obtain orange Preparation Example 15 (0.70 g, 94.8%). ¹H-NMR(400 MHz, , CDCl₃) δ 2.33 (s, 3H), 2.55 (t, J = 5.2 Hz, 4H), 3.37 (t, J= 5.2 Hz, 4H), 6.86 (d, J = 8.8 Hz,2H), 6.89 (d, J = 8.8 Hz, 2H), 7.38(d, J = 15.6 Hz, 1H), 7.49 (d, J = 8.8 Hz, 2H), 7.72 (d, J = 15.6 Hz, 1H), 7.95 (d, J = 8.8 Hz, 2H); ¹³C-NMR (100MHz, CDCl₃) 46.2, 47.4, 54.8, 113.7, 116.2, 118.8, 126.8, 128.7,130.3, 130.6, 143.7, 154.0, 159.7, 188.4 ppm.

### [Experimental Example 2] In silico 3D docking test

Binding ability of the Preparation Examples 1 to 15 to the AMP-activated protein kinase (AMPK) enzyme was identified.

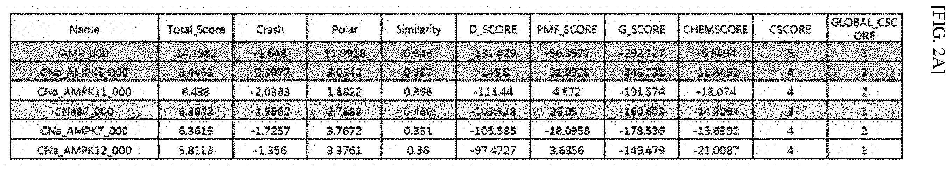

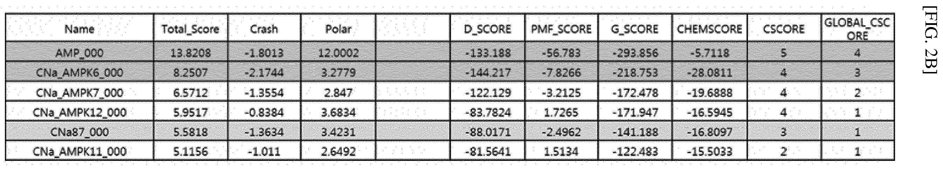

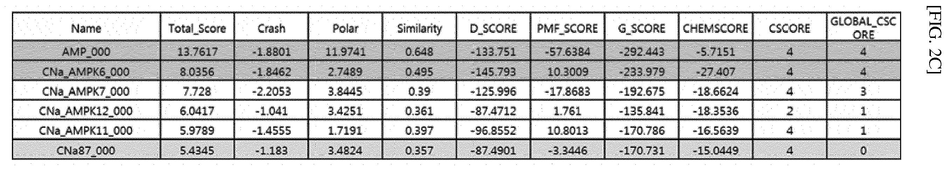

Using, as a control, the CNa87 molecule which is a mother body of the candidates, 3D docking experiments were carried out on the Preparation Examples 1 to 15, and the results are shown in Table 2 and FIG. 2.

As shown in Table 2 and FIG. 2, as compared with the control having a docking score of 5.5818, the Preparation Example 6 obtained by substitution with 4-chlorobenzenesulfonamide group exhibited the highest docking score of 8.2507; and the Preparation Examples 7 and 8 also exhibited a docking score which is about 1 or higher than the control.

**[Table 2]**

| Candidate | Docking score | Crash | Polarity |
|---|---|---|---|
| Control (CNa87) | 5.5818 | -1.3634 | 3.4231 |
| Preparation Example 1 | 5.9517 | -0.8384 | 3.6834 |
| Preparation Example 6 | 8.2507 | -2.1744 | 3.2779 |
| Preparation Example 7 | 6.5712 | -1.3554 | 2.847 |
| Preparation Example 8 | 7.8235 | -2.511 | 4.4773 |
| Preparation Example 10 | 5.4865 | -1.4533 | 2.798 |
| Preparation Example 11 | 5.1156 | -1.011 | 2.6492 |

From the above results, it can be seen that most of the above preparation examples exhibit high binding ability to the AMP-activated protein kinase enzyme as compared with the control CNa87, which makes it possible to predict that such preparation examples can activate the enzyme.

### [Experimental Example 2] Measurement of AMP-activated protein kinase activity

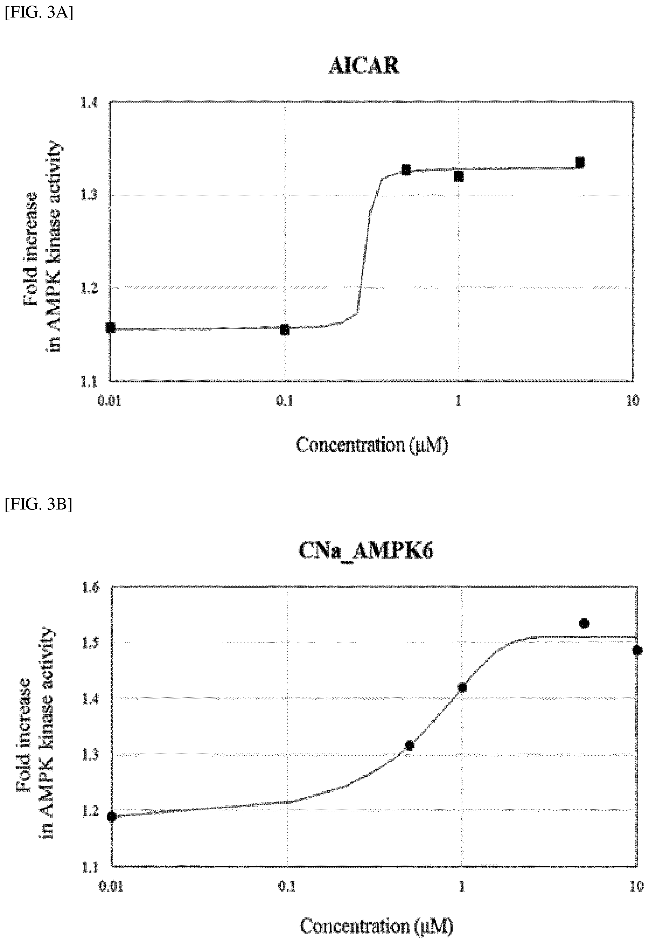

In order to identify an effect of the Preparation Example 6 having the highest docking score in Experimental Example 1 on activity of the AMP-activated protein kinase (AMPK), a kinase assay was performed. The kinase assay was performed as follows. The control AICAR, which is known to induce activation of the enzyme, and the Preparation Example 6, were administered. Then, purified AMP-activated protein kinase and ATP were allowed to react on a substrate peptide for the AMP-activated protein kinase, and degrees of luminescence thereof were shown as EC₅₀ in Table 3 and FIG. 3.

As shown in Table 3 and FIG. 3, the control AICAR exhibited an EC₅₀ of 0.299, while the Preparation Example 6 exhibited an EC₅₀ of 0.637 which is about 3-fold higher than the control.

**[Table 3]**

| Candidate | EC₅₀ (µM) |
|---|---|
| Control (AICAR) | 0.299 |
| Preparation Example 6 | 0.637 |

From the above results, it can be seen that the Preparation Example 6 according to the present invention not only exhibits high binding ability to the AMP-activated protein kinase, but also increases activity of the enzyme in a very effective manner.

### [Preparatory Example 11 Preparation and breeding of experimental animals

Six-week-old ICR mice (male) were purchased, and then examined for general health for 6 days while subjecting them to quarantine and acclimation under an environment of animal breeding room. Then, healthy individuals were selected and used for the experiment. Identification of each individual was made by marking the number of each individual on the tail using a permanent marker, and identification of a breeding box was made by attaching an individual identification card on which test number, test substance name, test item, date of acquisition, acclimation period, date of grouping, test period, sex/animal number, and person in charge are indicated. After excluding the individuals who developed abnormalities during the acclimation period and the individuals who did not gain weight normally, grouping was carried out so that uniform mean weight and standard deviation are achieved among respective groups.

A breeding environment for the experimental animals was such that the animals are bred while feeding food and drinking water in an animal breeding room which has been set at a breeding environment of temperature (22 ± 3 °C), relative humidity (50 ± 20) %, the number of ventilation (10 to 15) times/hour, lighting cycle of 12 hours (8:00 to 20:00), illumination (150 to 300) lux, and isolated breeding was carried out during the acclimation and test period.

### [Preparatory Example 2] Preparation of degenerative neurological disease-induced experimental animals

In order to induce a degenerative neurological disease, the experimental animals of Preparatory Example 1 were anesthetized by inhalation with 2.5% isoflurane. Then, 1 µg of amyloid-beta peptide₁₋₄₂, a degenerative neurological disease-inducing substance, was administered to the animals at both hippocampal positions (AP: -2.3, L: ±2.0, H: -1.5) using a stereotaxic apparatus. For the control, 1 µg of artificial cerebrospinal fluid made of 145 mM NaCl, 2.7 mM KCl, 1.2 mM CaCl₂, and 1.0 mM MgCl₂ was administered. For the administration, 1 µl was injected, at a flow rate of 0.2 µl/min, with a 1 ml gas-tight glass syringe (Hamilton, Reno, NV, USA) using a perfusion pump (Pump 22, Harvard Apparatus, South Natick, MA, USA). Subsequently, the syringe was allowed to stand for 5 minutes and then removed.

### [Experimental Example 3] Morris water maze test

A behavioral test was conducted to identify whether the compound according to the present invention has a therapeutic effect on degenerative neurological diseases.

Pre-treatment with donepezil, which is a positive control and a therapeutic agent for degenerative neurological diseases, and the Preparation Example 6 at 1mg, 10mg, and 100mg each was performed. Then, a degenerative neurological disease was induced as in Preparation Example 2. Donepezil and the Preparation Example 6 were administered continuously for 7 days, starting from a degenerative neurological disease-induction day until an experiment end day (anatomy day).

After induction of the degenerative neurological disease, the following test was conducted after a one-day recovery period passed.

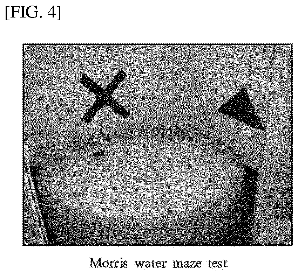

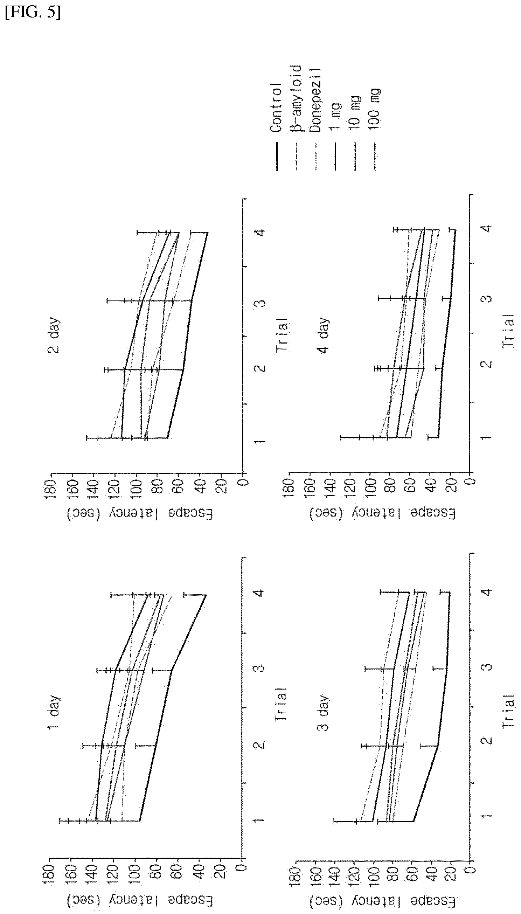

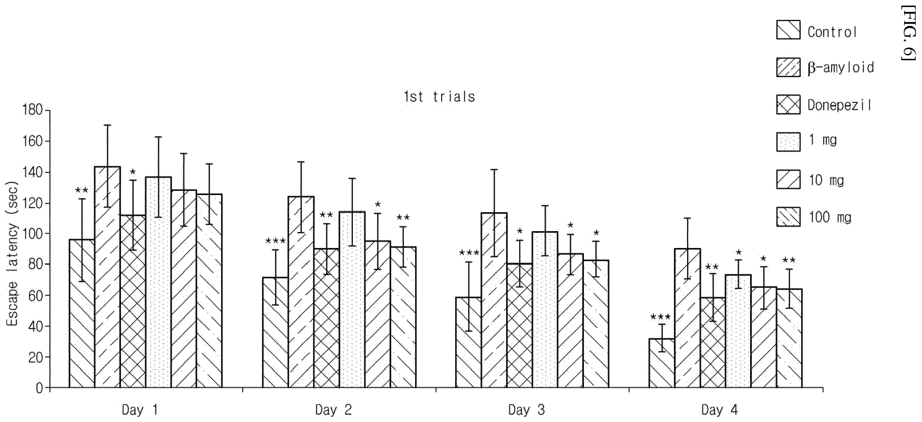

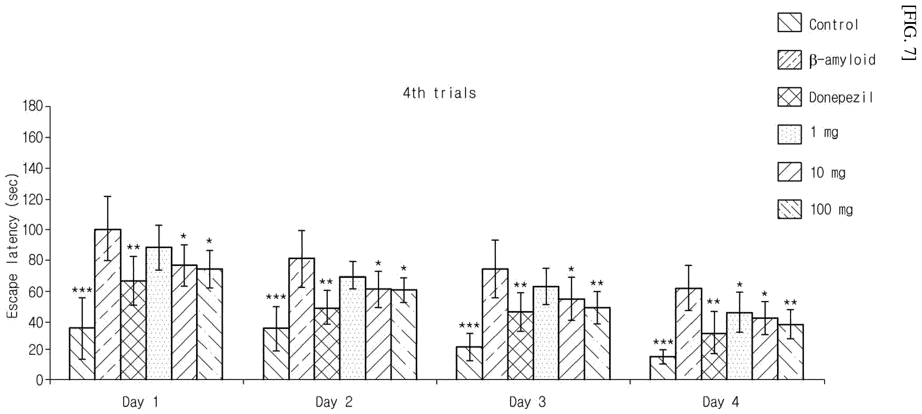

A circular pool (diameter: 120 cm, height: 75 cm) was filled with water to a depth of 25 ± 1 cm as in FIG. 4, and a 24 cm platform was prepared by hiding it 1 cm below the water surface. Space cues were installed on the four sides of the wall so that the aminals can identify locations, and skimmed milk powder was dissolved into water so that the animals cannot identify the platform with naked eyes. One day before the experiment, all the experimental animals were allowed to swim freely for 1 minute in a water bath having no platform, so that the animals can adapt to the swimming situation. After that, a test group was allowed to start, in any order, from two of the three quadrants except the quadrant where the platform was placed, and allowed to find the platform for 60 seconds. In a case where an animal finds the platform, the animal was allowed to stay thereon for 20 seconds. In a case where an animal does not find the platform within 45 seconds, the experimenter led the animal to the platform and caused the animal to stay thereon for 20 seconds. As soon as end of the position learning time, which is the 20-second staying time, the animal was allowed to start again from any quadrant, and the time and distance to reach the platform were measured. The procedure was performed four times a day, and all procedures were taken with a camera and analyzed with a video tracking system (Panlab, USA). Probe trials were made on the fifth day after the four-day experiment in toal was completed. The test group was allowed to swim freely for 90 seconds without the platform, thereby performing evaluation on whether the test group holds a memory of the platform location. The results are illustrated in FIGS. 5 to 7.

As illustrated in FIGS. 5 to 7, in a case of the control, on the first day, it found the platform at a level of 95.87 (± 26.88) seconds in the first trial. Learning progressed as the number of trials and the experimental days passed. In the first trials on respective experimental days, the control found the platform in 95.87 (± 26.88) seconds, 71.27 (± 17.83) seconds, 58.93 (± 22.16) seconds, and 32.37 (± 8.90) seconds which is on the last fourth day, respectively, and escaped. However, for the beta-amyloid group, which is a negative control, learning did not progress normally. The time taken to find the platform was 143.91 (± 26.88) seconds at the maximum on the first day, and 60.59 (± 15.12) seconds at the minimum on the fourth day. From this, it was identified that the escape latency is not decreased normally as compared with the control. The donepezil group found the platform in 111.99 (± 22.59) seconds at the maximum, and 30.82 (± 13.96) seconds at the minimum, and escaped.

On the other hand, for the groups treated with the Preparation Example 6 at 1, 10, and 100 mg, 136.45 (± 26.02) seconds, 128.59 (± 23.32) seconds, and 125.66 (± 19.35) seconds were respectively measured as the maximum platform escape latency. In addition, for the three test groups, 44.45 (± 13.35) seconds, 40.64 (± 10.94) seconds, and 36.48 (± 9.54) seconds were respectively measured as the minimum time to find the platform.

In addition, in a case where only the first trials on respective experimental days which are indicators showing a long-term memory after 24 hours, are compared among groups, on the first day, no significant difference was observable in all groups except the beta-amyloid group and the control. However, on the second and third days, it was possible to identify that the groups treated with the Preparation Example 6 at 10 mg (second day: 95.05 ± 18.06 seconds, p < 0.05, third day: 91.66 ± 12.68 seconds, p < 0.05), 100 mg (second day: 91.66 ± 12.68 seconds, p < 0.01, third day: 83.41 ± 11.46 seconds, p < 0.05) exhibit significantly decreased escape latency as compared with the beta-amyloid group (second day: 123.79 ± 22.85 seconds, third day: 113.10 ± 28.27 seconds). On the last fourth day, groups 1, 10, and 100 mg (1 mg: 73.58 ± 9.14 seconds, p < 0.05, 10 mg: 65.22 ± 13.65 seconds, p < 0.05, 100 mg: 64.7 ± 12.63 seconds, p < 0.05) exhibited significantly decreased escape latency as compared with the beta-amyloid group (90.39 ± 19.77 seconds). In addition, only the last fourth trials, which are indicators showing a short-term memory in repeated trials, were compared among groups. As a result, it was possible to identify that on the first, second, and third days, among the sample groups, 10 mg (first day: 76.14 ± 13.27 seconds, p < 0.05, second day: 59.87 ± 12.56 seconds, p < 0.05, third day: 54.07 ± 14.38 seconds, p < 0.05) and 100 mg (first day: 73.79 ± 12.15 seconds, p < 0.05, second day: 59.62 ± 8.22 seconds, p < 0.05, third day: 47.74 ± 10.81 seconds, p < 0.01) exhibit significantly decreased escape latency as compared with the beta-amyloid group (first day: 100.51 ± 21.51 seconds, second day: 80.39 ± 18.75 seconds, third day: 73.82 ± 19.18 seconds). In addition, on the last fourth day, all sample groups (1 mg: 44.45 ± 13.35 seconds, p < 0.05, 10 mg: 40.64 ± 10.94 seconds, p < 0.05, 100 mg: 36.48 ± 9.54 seconds, p < 0.01) exhibited significantly decreased escape latency as compared with the beta-amyloid group.

From the above results, it was found that the Preparation Example 6 recovers memory against degenerative dementia induced by beta-amyloid, thereby decreasing the escape latency.

### [Experimental Example 4] Probe test

In order to identify whether the compound according to the present invention has a therapeutic effect on degenerative neurological diseases, comprehensive memory ability was tested.

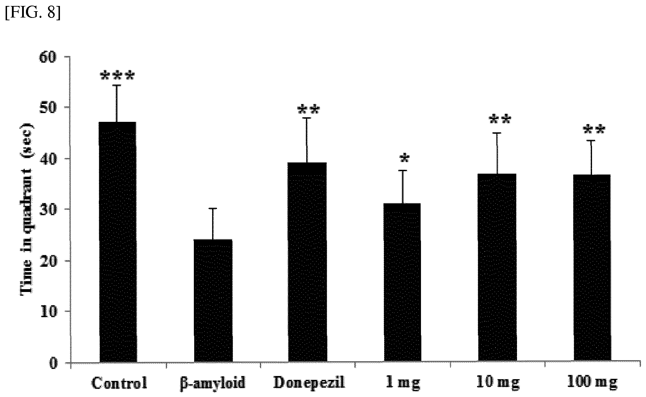

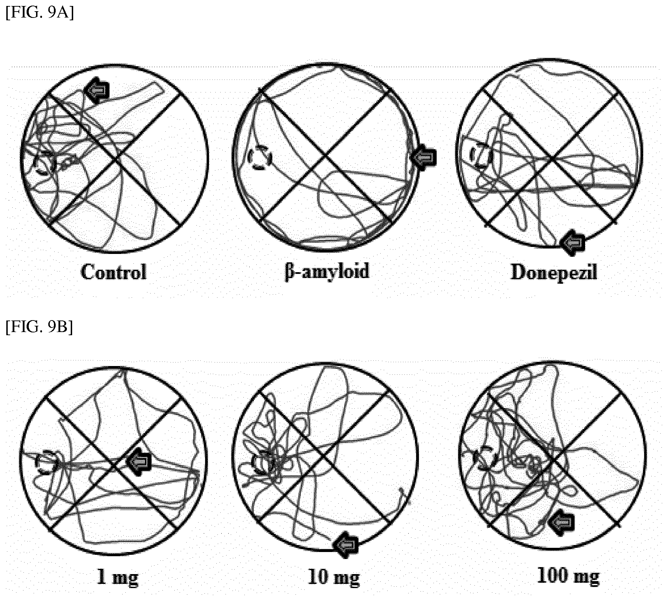

In the same manner as in Example 3, the Preparation Example 6, the negative control, and donepezil as a positive control were respectively administered, and a probe test was conducted to evaluate comprehensive final memory ability through 4-day repetitive learning. The results are illustrated in FIGS. 8 and 9.

As illustrated in FIGS. 8 and 9, from the probe test results, it was identified that in a case where the platform is removed, for 90 seconds, the control has the swimming time of 47.19 (± 6.95) seconds in the quadrant where the platform has been placed, and the beta-amyloid group has the swimming time of 23.90 (± 6.25) seconds in the quadrant where the platform has been placed, which is significantly decreased as compared with the control. The donepezil group, which is a positive control, had the swimming time of 39.04 (± 8.68) seconds in the target quadrant, which is significantly increased as compared with the beta-amyloid group (p < 0.01). In the test groups, it was identified that all groups (1 mg: 31.08 ± 6.33 seconds, 10 mg: 36.73 ± 7.78 seconds, 100 mg: 36.51 ± 6.55 seconds) exhibit significantly increased swimming time in the target quadrant as compared with the beta-amyloid group. (1 mg: p < 0.05, 10, 100 mg: p < 0.01).

### [Experimental Example 5] Passive avoidance response test

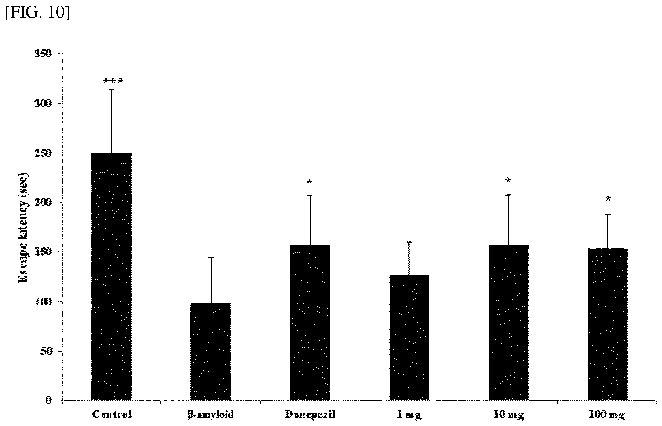

In the same manner as in Example 3, the Preparation Example 6, the negative control, and donepezil as a positive control were respectively administered. Under a situation where a chamber with a bright light and a dark chamber were connected to each other via a small passage, 4 hours before the experiment, the experimental animals were placed in the chamber with a bright light. The experimental animals instinctively enter the dark chamber. However, after 4 hours of fear learning carried out by using an electric shock to give aversive stimulus, in a case where the experimental animals are placed in the chamber with a bright light, the time until the experimental animals enter the dark chamber was measured and quantified. The results are illustrated in FIG. 10.

As illustrated in FIG. 10, the above experimental results show that for the control, the time taken to enter the dark chamber starting from the chamber with a bright light was measured as 249.84 (± 64.84) seconds, and for the beta-amyloid group, the time was 98.21 (± 43.27) seconds, indicating that the beta-amyloid group exhibits significantly decreased staying time in the chamber with a bright light as compared with the control. In addition, it was identified that among the test groups, groups 10 mg (156.87 ± 50.95 seconds) and 100 mg (153.48 ± 34.71 seconds) exhibit significantly increased staying time in the chamber with a bright light as compared with the beta-amyloid group (p < 0.05).

### [Experimental Example 6] Rotarod test

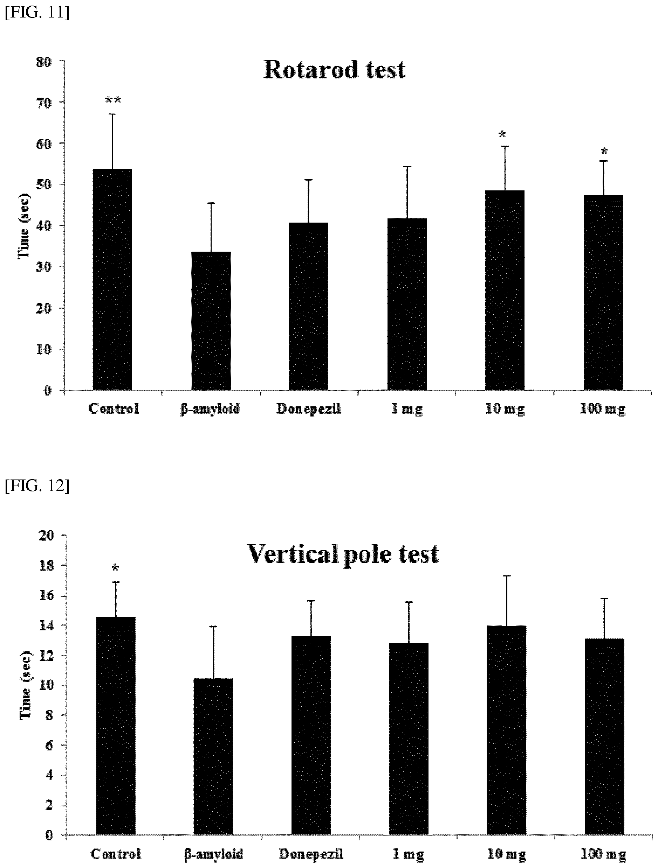

In the same manner as in Example 3, the Preparation Example 6, the negative control, and donepezil as a positive control were respectively administered. As an experiment for evaluating effects of the samples on decreased motor coordination ability and motor sensation associated with a degenerative neurological disease, a rotarod experiment which examines the overall motor function of the test groups was conducted. The experimental method was as follows. On the day before the experiment, the animals were subjected to adaptive training by being forced to walk on a rotating (20 rpm) cylinder twice for one minute each time. After 24 hours, the time to lose balance and fall off the rotating cylinder of the same condition was measured, and the result is illustrated in FIG. 11.

As illustrated in FIG. 11, for the control, the time to keep balance on the cylinder rotating at 15 rpm was 53.62 (± 13.59) seconds on average, and for the beta-amyloid group, the time was 33.60 (± 11.95) seconds which is significantly decreased as compared with the control. In the test groups, it was possible to identify that groups 10 mg (48.62 ± 10.54 seconds) and 100 mg (47.65 ± 8.13 seconds) exhibit significantly increased time on the cylinder as compared with the beta-amyloid group (p < 0.05).

### [Experimental Example 7] Vertical pole test

In the same manner as in Example 3, the Preparation Example 6, the negative control, and donepezil as a positive control were respectively administered. Among effects of the samples on decreased motor coordination ability and motor sensation associated with a degenerative neurological disease, in order to evaluate sensation of balance, experimental animals were placed on the center of a pole inclined at an angle of 45°, and then the time taken to lose balance and fall was measured. The results were illustrated in FIG. 12.

As illustrated in FIG. 12, in a case of the control, the time to keep balance on the pole inclined at an angle of 45° was 14.60 (± 2.28) seconds on average; and in a case of the beta-amyloid group, the time was significantly decreased to 10.47 (± 3.50) seconds.

### [Experimental Example 8] Measurement of acetylcholine hydrolase activity

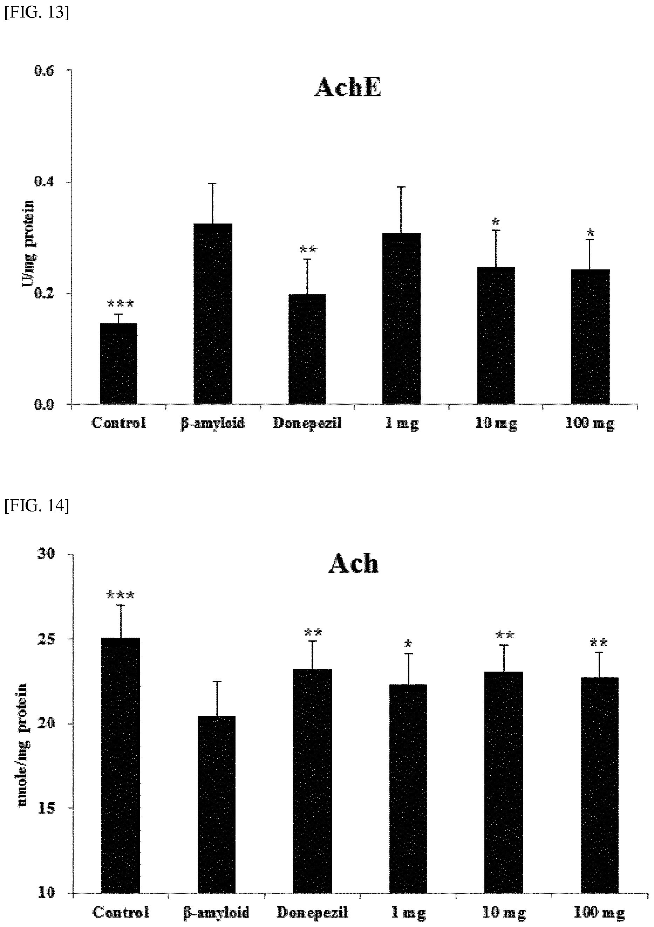

Experimental mice were respectively administered the Preparation Example 6, the negative control, and donepezil as a positive control in the same manner as in Example 3, and the brain tissues were extracted therefrom. Then, acetylcholine hydrolase activity was measured by the following method. To a microplate were continuously added 300 µl of 0.1 M Tris buffer, pH 8.0 (Trizma HCl + Trizmabase), 20 µl of 0.01 M dithionitrobenzoic acid (DTNB; Sigma, USA), 10 µ1 of brain tissue homogenate (enzyme suspension). Immediately before absorbance measurement, 10 µl of 0.1 M acetylthiocholine chloride (Sigma, USA) as a substrate was added thereto. An absorbance meter was used to observe absorbance changes at 405 nm for 5 minutes, so that acetylcholine hydrolase activity (unit/min/mg protein) was measured. The results are illustrated in FIG. 13.

As illustrated in FIG. 13, the control exhibited an activity level of 0.15 U/mg protein (± 0.02); however, the beta-amyloid group exhibited activity of 0.31 U/mg protein (± 0.06) which is a significant difference of about 2.2-fold increase rate. On the other hand, it was identified the donepezil group, which is a positive control, exhibits 0.18 U/mg protein (± 0.20), indicating significantly decreased acetylcholine hydrolase activity as compared with the beta-amyloid group. Among the sample groups, the groups 10 mg (0.25 ±0.07 U/mg protein) and 100 mg (0.24 ± 0.05 U/mg protein) exhibited significantly decreased enzyme activity.

### [Experimental Example 9] Measurement of acetylcholine content

In the same manner as in Example 3, the Preparation Example 6, the negative control, and donepezil as a positive control were respectively administered. Measurement of acetylcholine in brain tissues was performed based on response of o-acyl derivatives with alkaline hydroxylamine. Specifically, 50 µl of brain tissue was taken and 50 µl of 1% hydroxylamine (Sigma, USA) was added and mixed. Then, pH thereof was adjusted to 1.2 ± 0.2 using hydrochloric acid. Finally, 500 µl of FeCl₃ (10% in 0.1 N HCl) was added to measure an acetylcholine level (umole/mg protein), and then the absorbance was measured at 530 nm. The results are illustrated in FIG. 14.

As illustrated in FIG. 14, according to the measurement results obtained by the above method, the control showed a content of 25.07 µmole/mg protein (± 1.92). The beta-amyloid group showed a content of 20.48 µmole/mg protein (± 2.05) which is a significant difference and corresponds to a 1.25-fold decrease. However, it was identified that the donepezil group, which is a positive control, shows 23.19 µmole/mg protein (± 1.69), indicating a significantly increased content as compared with the beta-amyloid group. It was identified that the measured values in all sample groups are 1 mg: 22.31 ± 1.80 µmole/mg protein, 10 mg: 23.08 ± 1.54 µmole/mg protein, and 100 mg: 22.79 ± 1.45 µmole/mg protein, indicating a significantly increased content as compared with the beta-amyloid group (1 mg: p < 0.05, 10, 100 mg: p < 0.01).

From the above results, it can be seen that the Preparation Example 6 according to the present invention remarkably decreases an acetylcholine content even in a case of being compared with donepezil, a positive control.

### [Experimental Example 10] Measurement of apoptosis level

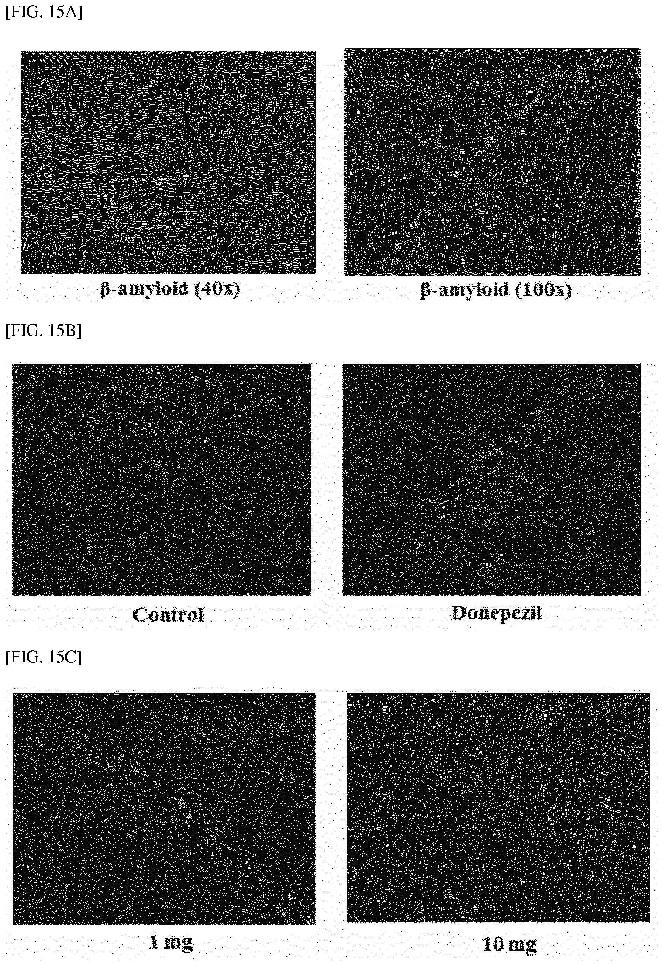

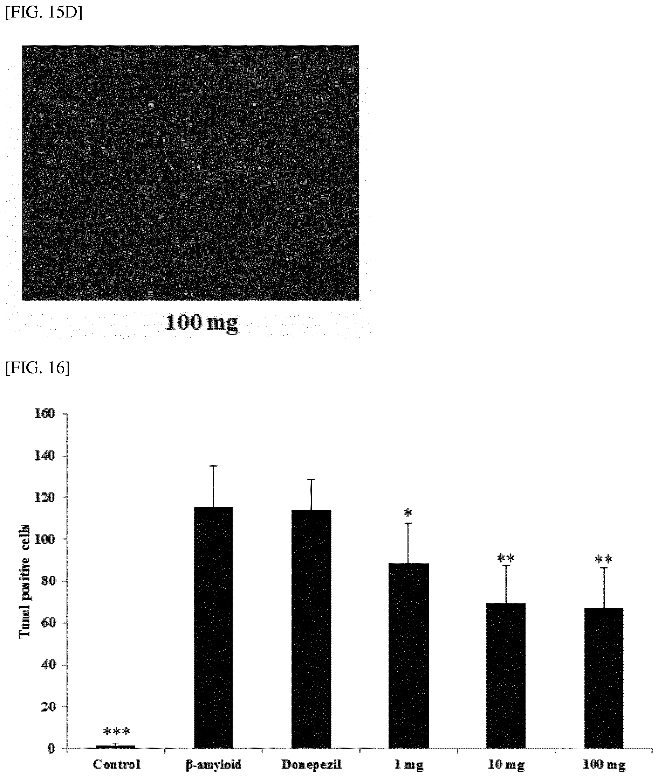

Experimental mice were respectively administered the Preparation Example 6, the negative control, and donepezil as a positive control in the same manner as in Example 3, and the brain tissues were extracted therefrom. Then, an apoptosis level was checked through TUNEL assay. The TUNEL assay is a method to search for cells that have undergone apoptosis by attaching a fluorescent substance to ends of DNA fragments cleaved due to apoptosis. The extracted brain tissue of the experimental animal is fixed by perfusion with 4% formaldehyde. Then, the brain tissue was embedded in paraffin and sectioned to a thickness of 5 um. The brain tissue fixed on a slide was subjected to a TUNEL assay kit so that the end portions where DNA having undergone apoptosis had been fragmented are fluorescence-stained, and checked by a fluorescence microscope. The results are illustrated in FIGS. 15 and 16.

As illustrated in FIGS. 15 and 16, as a result of conducting the TUNEL assay for all experimental groups in Table 3, the number of Tunel positive cells was observed as 1.1 (± 1.32) on average in the control; and the number of Tunel positive cells was significantly increased to 115.6 (± 19.52) in the beta-amyloid group. The donepezil group (113.83 ± 14.77), which is a positive control, had no significant change. However, it was identified that all sample groups (1 mg: 88.83 ± 18.98, 10 mg: 69.5 ± 17.87, 100 mg: 67.00 ± 19.54) exhibit a significantly decreased number of Tunel positive cells as compared with the beta-amyloid group (1 mg: p < 0.05, 10, 100 mg: p < 0.01).

From the above results, it can be seen that the Preparation Example 6 according to the present invention remarkably decreases apoptosis in the brain tissue even in a case of being compared with donepezil, a positive control.

### [Experimental Example 11] Physical property evaluation: Measurement of membrane permeability

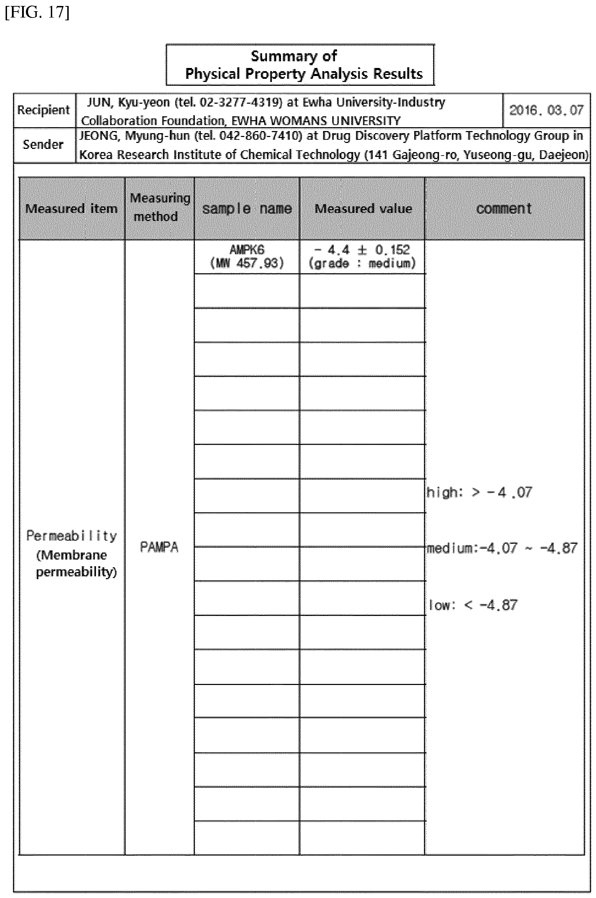

In order to measure a degree of membrane permeability of the Preparation Example 6 according to the present invention, measurement was performed by the PMPA method in which an artificial lipid membrane is formed and permeability is measured by a method other than cells. The results are illustrated in FIG. 17.

As illustrated in FIG. 17, it was found that the Preparation Example 6 according to the present invention exhibits a membrane permeability measured value of -4.4 ± 0.152, indicating a medium value in term of degree of permeability.

From the above results, it can be seen that the Preparation Example 6 according to the present invention has membrane permeability and thus may be indeed effectively utilized clinically.

### [Experimental Example 12] Physical property evaluation: Measurement of mutagenicity

For cancer-causing potential due to the Preparation Example 6 according to the present invention, measurement was performed by the Ames experimental method in which mutagenicity of a test substance is predicted through a mutation reaction test such as substitution, addition, deletion of a few DNA bases using Salmonella typhimurium strains that require a specific amino acid, and thus toxicity thereof is measured.

In the Ames experimental method, the Preparation Example 6, and 2-nitrofluorene, benzo(a)pyrene, and sodium azide which correspond to the positive controls were respectively dissolved and diluted in DMSO, and treatment with the resultants was performed. Using Salmonella typhimurium strains TA98 and TA100, with or without S9, a degree of bacterial growth depending on the test substance was observed in the control not treated with the Preparation Example 6 and the test group treated therewith. For the Preparation Example 6, a preliminary test with 6-level concentrations obtained by dilution at 1/5 starting from 5000 µg/plate, which is the highest treatment concentration in the Ames experimental method, was conducted to select an appropriate concentration that does not exhibit a bacterial growth inhibition phenomenon and toxicity, and a concentration that does not exhibit precipitation during treatment. For each strain, treatment with a single concentration of 200 µg/plate was performed in triplicate and mutation was checked. The results are shown in Table 4 below.

**[Table 4]**

| Test strain | Compound | Concentration | Reverse mutation/dish (mean±SD) [factor] | |
|---|---|---|---|---|
| | | | w/o S-9 mix | with S-9 mix |
| TA98 | Control | 0 | 19±2 | 23±2 |
| | Preparation Example 6 | 200 | 20±4 [1.1] | 35±2 [1.5] |
| TA100 | Control | 0 | 112±16 | 111±5 |
| | Preparation Example 6 | 200 | 104±12 [0.9] | 117±4 [1.0] |
| TA98 | 2-Nitrofluorene | 2 | 290±8 915.0] | Not available |
| | Benzo(a)pvrene | 2 | Not available | 184±17 [8.0] |
| TA100 | Sodium azide | 1 | 618±13 [5.5] | Not available |
| | Benzo (a)pvrene | 2 | Not available | 567±23 [5.1] |

As shown in Table 4, in the TA98 and TA100 strains, in a case where no metabolic activation enzyme system is applied and in a case where the metabolic activation enzyme system is applied, the concentration groups, in which treatment with the Preparation Example 6 at 200 µg/plate each had been performed, did not show a significant increase in reverse mutation as compared with the control. All positive controls exhibited a significantly increased number of colonies as compared with the control. The prepared test substance and the S9 mix were each mixed in the upper agar and culture was performed for 48 hours. As a result, contamination was not observed.

From the Ames experiment's results which are reliable in view of the fact that the above results show a remarkable difference while showing a 3-fold or higher increase in the number of colonies as compared with the control, it is determined that the Preparation Example 6 is negative since no increase in the number of colonies is observed in a reverse mutation test conducted for bacteria obtained by treating the Salmonella typhimurium TA98 and TA100 strains with the Preparation Example 6. Thus, it can be expected that the Preparation Example 6 according to the present invention is very unlikely to cause cancer.

### [Experimental Example 13] Physical property Test: Cytotoxicity test

In order to identify whether the Preparation Example 6 according to the present invention is toxic to cells, a cytotoxicity test was conducted at the Korea Research Institute of Chemical Technology using the Cyto X™ cell viability assay kit (LPS solution). The results are shown in Table 5 below.

As shown in Table 5, it can be determined that the Preparation Example 6 has no cytotoxicity, because it can be generally predicted that a compound has no cytotoxicity in a case where IC₅₀ by the compound is 10 µM or higher.

**[Table 5]**

| Compound | IC₅₀ (µM) | | | | |
|---|---|---|---|---|---|
| | VERO | HFL-1 | L929 | NIH 3T3 | CHO-K1 |
| Preparation Example 6 | 83.6 | >100 | >100 | >100 | >100 |

From the above results, it can be expected that the Preparation Example 6 according to the present invention does not exhibit cytotoxicity in a case of being used in the form of a pharmaceutical composition for preventing or treating degenerative neurological diseases.

### [Experimental Example 14] Physical property test: Cardiac stability test

Cardiac stability of the Preparation Example 6 according to the present invention was checked using a ligand binding assay. The results are shown in Table 6 below.

In the ligand binding assay, in a case where an inhibition rate by a 10 µM compound is 50 or higher, it is determined that the substance requires attention for binding in hERG channel. As shown in Table 6, it was identified that the Preparation Example 6 exhibits an inhibition rate of 1 or lower per 10 µM, and thus would be safe for protein binding in hERG channel.

**[Table 6]**

| Treatment | Concentration (µM) | Inhibition rate (%) |
|---|---|---|
| Control | 10 | 69.4±4.78 |
| Preparation Example | 10 | < 1 |

From the above results, it can be expected that the Preparation Example 6 according to the present invention has cardiac stability in a case of being used in the form of a pharmaceutical composition for preventing or treating degenerative neurological diseases.

### [Experimental Example 15] Organic physical property test

For a preliminary examination on clinical application of the Preparation Example 6, evaluation on lipophilicity and solubility was performed. The results are shown in Table 7 below.

As shown in Table 7 below, the Preparation Example 6 was measured to have lipophilicity of 4.75, and solubility of 206.2 and 94.4.

**[Table 7]**

| Measured item | Method | Measured value | Remark |
|---|---|---|---|
| pKa, LogP, | ACD/Labs | 4.75 | |
| lipophilicity | T3 (pH-metrior) | | |
| Solubility | Nephelometry | 206.2±1.7µM/94.4±0.8µg/ml | DMSO 5%/water solvent |

From the above results, it can be seen that the Preparation Example 6 according to the present invention has organic physical properties which make it easy to be used in the form of a pharmaceutical composition for preventing or treating degenerative neurological diseases.

### [Experimental Example 16] Physical property test: Half-life

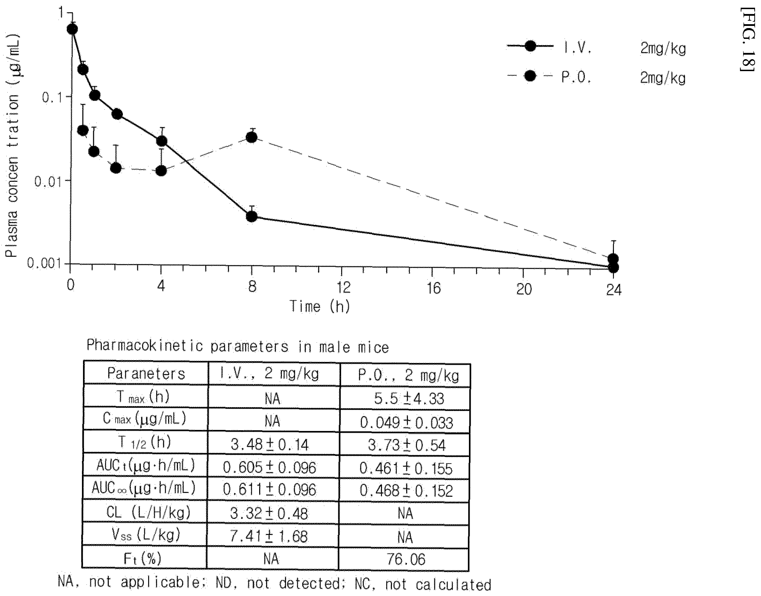

In order to identify whether the Preparation Example 6 according to the present invention can be orally administered, a half-life of the compound was checked. A request for the half-life test was made to the Korea Research Institute of Chemical Technology, and comparison was made between the half-life of the compound and the half-life of Resveratrol as a control. The results are illustrated in FIG. 18.

As illustrated in FIG. 18, Resveratrol as a control has a very short half-life of 8 to 10 minutes, while the Preparation Example 6 according to the present invention has a half-life corresponding to 3 hours or longer.

The above results show that the Preparation Example 6 according to the present invention has a relatively long half-life even *in vivo,* which enables treatment of degenerative neurological diseases via oral administration.

Although the present invention has been described in detail above, the scope of the present invention is not limited thereto. It will be obvious to those skilled in the art that various modifications and changes can be made without departing from the technical spirit of the present invention described in the claims.

### Industrial Applicability

A novel compound of the present invention and a pharmaceutical composition comprising the same as an active ingredient induce AMP-activated protein kinase (AMPK) enzyme activation, and thus may be usefully utilized in the fields associated degenerative neurological diseases and the like.

## Claims

1. A compound represented by the following Formula 1: 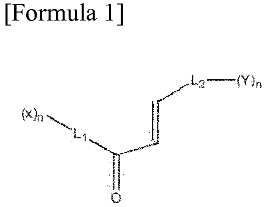 in the Formula 1,
L₁ to L₂ are each independently selected from the group consisting of C₃ to C₄₀ cycloalkylene, C₆ to C₆₀ arylene, heteroarylene having 5 to 60 nuclear atoms;
X and Y are each independently selected from the group consisting of deuterium, halogen, cyano, nitro, sulfonyl, C₁ to C₁₀ alkylsulfonyl, azide, hydroxy, C₁ to C₄₀ alkyl, C₂ to C₄₀ alkenyl, C₁ to C₄₀ alkoxy, unsubstituted or substituted C₆ to C₆₀ aryloxy, unsubstituted or substituted C₃ to C₄₀ cycloalkyl, unsubstituted or substituted heterocycloalkyl having 3 to 20 nuclear atoms, unsubstituted or substituted C₆ to C₆₀ aryl, unsubstituted or substituted heteroaryl having 5 to 60 nuclear atoms, and -NR'R";
R' and R" are each independently selected from the group consisting of hydrogen, C₁ to C₁₀ alkyl, C₆ to C₆₀ aryl, C₃ to C₄₀ cycloalkyl, C₆ to C₆₀ arylsulfonyl, heteroaryl having 5 to 60 nuclear atoms; and
n is selected from integers of 0 to 5.

2. The compound according to claim 1,
wherein L₁ and L₂ are C₆ to C₆₀ arylene.

3. The compound according to claim 1,
wherein L₁ and L₂ are phenylene;
n is an integer of 1 or 2; and
X and Y are the same as or different from each other and are each independently selected from the group consisting of sulfonyl, C₁ to C₁₀ alkylsulfonyl, C₁ to C₄₀ alkoxy, -NR'R", hydroxy, C₆ to C₆₀ aryloxy, and unsubstituted or substituted heterocycloalkyl having 3 to 20 nuclear atoms.

4. The compound according to claim 3,
wherein X is -NR'R"; and
R' and R" are the same as or different from each other and are each independently hydrogen or a substituent of the following Formula 2: 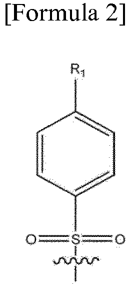 in the formula, R₁ is selected from the group consisting of hydrogen, deuterium, halogen, and nitro.

5. The compound according to claim 3,
wherein Y is C₁ to C₆ alkoxy.

6. The compound according to claim 3,
wherein Y is selected from the following Formula 3 or Formula 4: 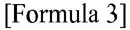 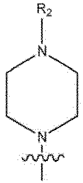 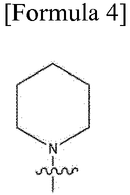 in the formulas, R₂ is selected from the group consisting of hydrogen, deuterium, halogen, C₁ to C₄₀ alkyl, and C₂ to C₄₀ alkenyl.

7. The compound according to claim 1,
wherein the compound is selected from the group consisting of the following compounds: 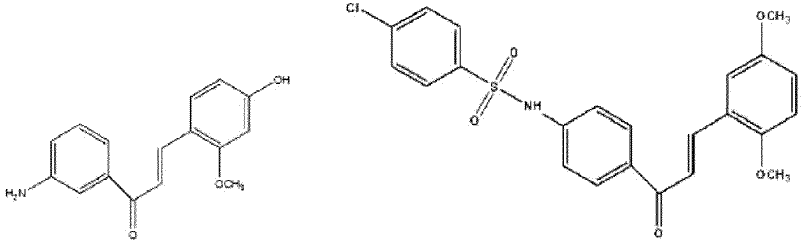 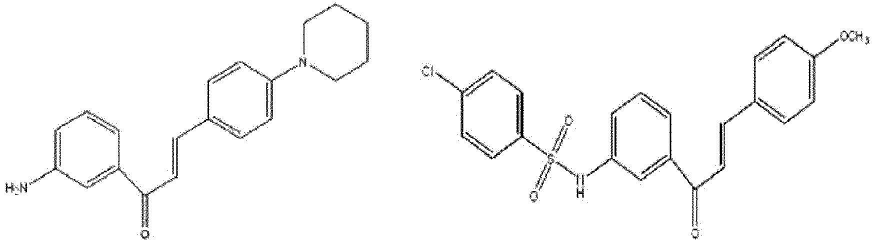 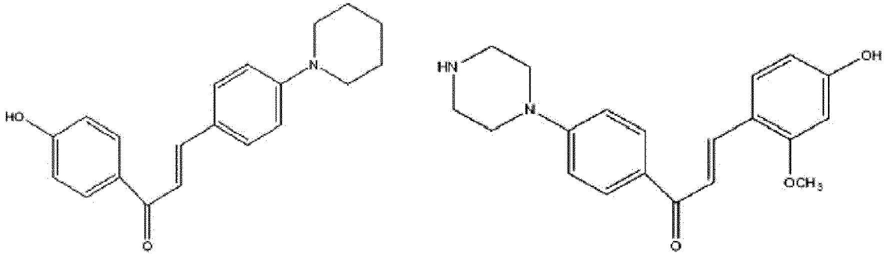 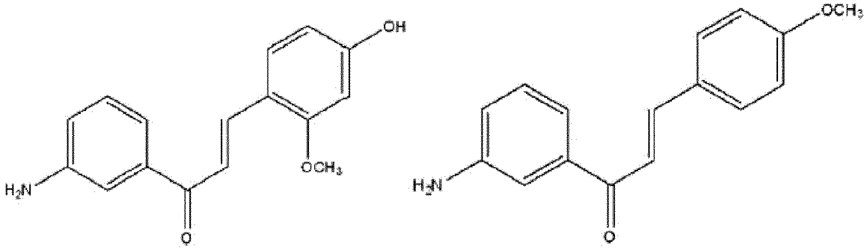 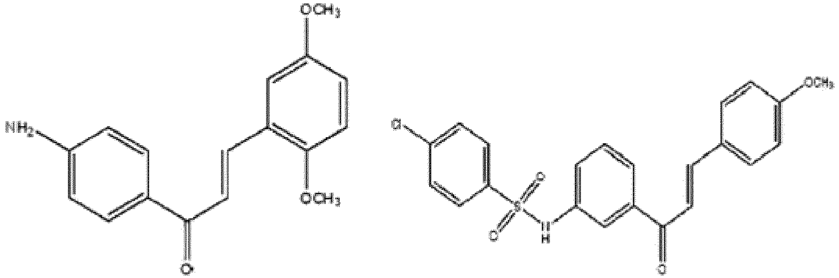 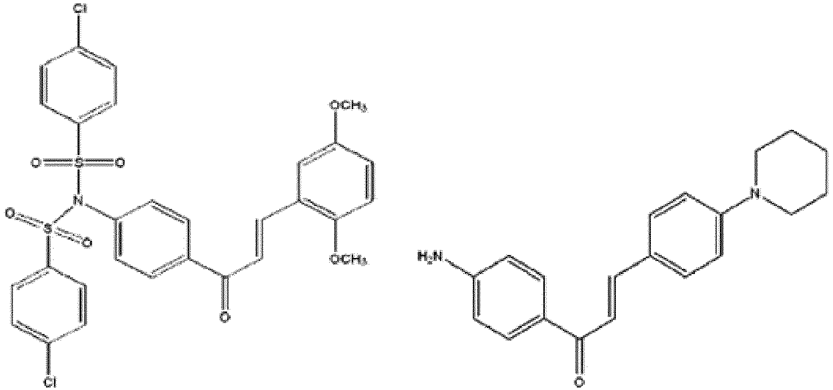 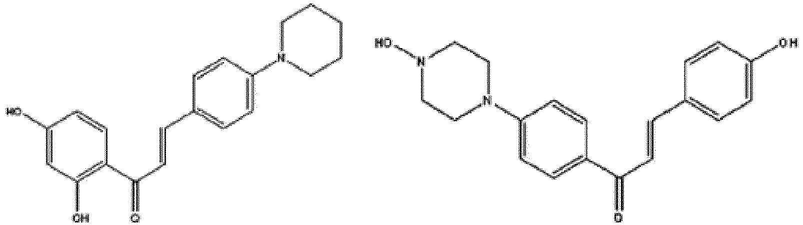 and

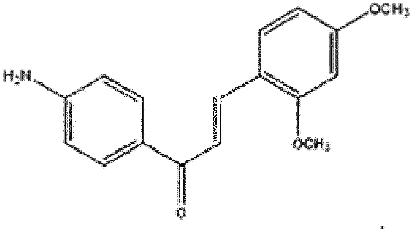

8. A method for preparing the compound according to claim 1, comprising:
1) a step of mixing an acetophenone derivative and a benzaldehyde derivative with an organic solvent, and performing stirring; and
2) a step of extracting the reactant of the step 1) using an organic solvent.

9. The method according to claim 8,
wherein the acetophenone derivative is 3-aminoacetophenone or 4-aminoacetophenone.

10. The method according to claim 8,
wherein the benzaldehyde derivative is selected from any one of the compound 2,4-dimethoxybenzaldehyde, 2,5-dimethoxybenzaldehyde, 4-methoxybenzaldehyde, 2-methoxy-4-((tetrahydro-2H-pyran-2-yl)oxy)benzaldehyde, or 4-(piperidin-1-yl)benzaldehyde.

11. The method according to claim 8, further comprising, after the step 2), a step of adding a substituted alkylsulfonyl chloride-based compound and a base, and performing mixing and stirring.

12. A pharmaceutical composition for preventing or treating degenerative neurological diseases, comprising as an active ingredient:
the compound of any one of claims 1 to 7.

13. The pharmaceutical composition according to claim 12,
wherein the degenerative neurological disease is one or more selected from the group consisting of stroke, palsy, dementia, Alzheimer's disease, Huntington's disease, amyotrophic lateral sclerosis (ALS), Pick's disease and Creutzfeldt-Jakob disease.

14. The pharmaceutical composition according to claim 12,
wherein the degenerative neurological disease is a disease caused by brain neuron damage resulting from amyloid-beta peptide-induced stress.

15. The pharmaceutical composition according to claim 12,
wherein the composition induces the AMP-activated protein kinase (AMPK) enzyme activation.
